(19) Europäisches Patentamt / European Patent Office / Office européen des brevets

(11) **EP 4 722 710 A1**

(12) **EUROPÄISCHE PATENTANMELDUNG**

(43) Veröffentlichungstag:
**08.04.2026 Patentblatt 2026/15**

(21) Anmeldenummer: 25203060.6

(22) Anmeldetag: **18.09.2025**

(51) Internationale Patentklassifikation (IPC):
**G01N 29/07** (2006.01)   **G01N 29/04** (2006.01)
**G01N 29/265** (2006.01)   **G01N 29/28** (2006.01)
**G01N 29/44** (2006.01)   *G01N 33/38* (2006.01)

(52) Gemeinsame Patentklassifikation (CPC):
**G01N 29/07; G01N 29/043; G01N 29/265;**
**G01N 29/28; G01N 29/4427;** G01N 33/383;
G01N 2291/011; G01N 2291/0232;
G01N 2291/0258; G01N 2291/048;
G01N 2291/2698

(84) Benannte Vertragsstaaten:
**AL AT BE BG CH CY CZ DE DK EE ES FI FR GB GR HR HU IE IS IT LI LT LU LV MC ME MK MT NL NO PL PT RO RS SE SI SK SM TR**
Benannte Erstreckungsstaaten:
**BA**
Benannte Validierungsstaaten:
**GE KH LA MA MD TN**

(30) Priorität: **01.10.2024 DE 102024128444**

(71) Anmelder: **Bundesrepublik Deutschland (Bundesanstalt für Materialforschung und -prüfung) 12205 Berlin (DE)**

(72) Erfinder:
• **PIRSKAWETZ, Stephan**
  **12305 Berlin (DE)**
• **HÜSKEN, Götz**
  **12349 Berlin (DE)**

(74) Vertreter: **Zimmermann & Partner Patentanwälte mbB P.O. Box 330 920 80069 München (DE)**

(54) **AUTOMATISIERTE ULTRASCHALLPRÜFUNG VON SPANNBETONSCHWELLEN**

(57)    Es wird ein zerstörungsfreies Prüfverfahren zur Beurteilung der Einsetzbarkeit einer Spannbetonschwelle (1) als Gleisschwelle in einem Gleisbett für ein Eisenbahnschienennetz vorgeschlagen, aufweisend: Anordnen (1100) der zu prüfenden Spannbetonschwelle (1) in einem Ankopplungsfluid (2); Positionieren (1200) eines Paares von Ultraschallprüfköpfen (3) beidseitig der zu prüfenden Spannbetonschwelle (1) in einem jeweiligen definierten Abstand (lc1 , lc2 ) und in einem jeweilig definierten Winkel zu Oberflächen der Spannbetonschwelle (1) in mindestens einer Messposition; Aussenden (1310) eines Ultraschallsignals durch einen als Sender (3.1) konfigurierten Ultraschallprüfkopf des Paares von Ultraschallprüfköpfen (3) und Empfangen (1320) eines Durchschallungssignals durch einen als Empfänger (3.2) konfigurierten Ultraschallprüfkopf des Paares von Ultraschallprüfköpfen (3), wobei das Aussenden (1310) und das Empfangen (1320) in der mindestens einen Messposition erfolgt; Ermitteln (1400) einer Ultraschalllaufzeit ($t_s$) des in der mindestens einen Messposition ausgesendeten und empfangenen Ultraschallsignals im Beton der Spannbetonschwelle (1); und Bewerten (1700) der Spannbetonschwelle (1) als tauglich für die Verwendung als Gleisschwelle im Gleisbett, wenn die ermittelte Ultraschalllaufzeit in jeder Messposition der mindestens einen Messposition unterhalb eines jeweiligen Schwellenwertes liegt oder dem jeweiligen Schwellenwert entspricht, und als untauglich für die Verwendung der Spannbetonschwelle (1) als Gleisschwelle im Gleisbett, wenn die ermittelte Ultraschalllaufzeit in wenigstens einer Messposition der mindestens einen Messposition oberhalb des jeweiligen Schwellenwertes liegt. Weiter wird ein System (100, 110, 120, 130) zur Prüfung und/oder Zertifizierung einer Spannbetonschwelle (1) mit dem bezeichneten zerstörungsfreien Prüfverfahren (1000) vorgeschlagen, sowie dessen verfahrensgerechte Verwendung sowie die Wiederverwendung einer im zerstörungsfreien Prüfverfahren (1000) als tauglich befundenen Spannbetonschwelle (1) in einem Gleisbett vorgeschlagen.

**(Forts. nächste Seite)**

120

FIG. 5

**Beschreibung**

TECHNISCHES GEBIET

Die vorliegende Offenbarung betrifft die zerstörungsfreie Prüfung von Spannbetonschwellen für den Bahn- und Schienenverkehr.

TECHNISCHER HINTERGRUND

Für eine Bewertung der Qualität von Spannbetonschwellen, beispielsweise im Rahmen von Zulassungsprüfungen und Zertifizierungen oder bei einer Qualitätsprüfung auf Wiederverwendbarkeit zuvor bereits genutzter und/oder aufbereiteter Spannbetonschwellen, kommt deren Beurteilung hinsichtlich des Fehlens von Brüchen, Ausbrüchen, Rissen und/oder Hohlräumen als objektiv messbaren Entscheidungskriterien eine große Bedeutung zu.

Vorbekannte Prüfverfahren basieren typischerweise auf einer fachmännischen Inaugenscheinnahme, bzw. visuellen Begutachtung.

Die visuelle Prüfung weist jedoch eine Reihe von Nachteilen auf. Diese Art der Prüfung ist aufwendig, per se subjektiv und damit unzuverlässig, zumal keine qualifizierten Anforderungen an das die Prüfung durchführende Personal gestellt werden. Ein aus CA 2 955 105 A1 vorbekannter Ansatz, eine visuelle Prüfung zu objektivieren und vor allem, die Empfindlichkeit der Prüfung zu steigern basiert auf der Messung einer Ausbreitungsgeschwindigkeit eines Impulses entlang einer im Gleisbett angeordneten Betonschwelle. Auch in KR 10 1027 910 B1 ist ein automatisches Prüfverfahren zur Erkennung von Rissen in einer im Gleisbett angeordneten Eisenbahnschwelle durch akustische Signale offenbart. Ein auf der Reflektion von Ultraschallsignalen basierendes bildgebendes Verfahren für eine einzelne, dem Gleisbett entnommene Betonschwellen ist von Strangfeld und Küttenbaum (2023) in dem wissenschaftlichen Beitrag "Zur Visualisierung von Schäden in Betonschwellen durch zerstörungsfreie Ultraschallprüfung" in Eisenbahntechnische Rundschau 12/23: 26-31 beschrieben.

Vorbekannte Lösungen sind jedoch entweder auf die im Gleisbett angeordnete Schwelle gerichtet und damit ohnehin nur bedingt aussagefähig oder zu zeit- und kostenaufwendig, um eine zuverlässige Aussage über die Wiederverwendbarkeit einer gebrauchten Spannbetonschwelle bei einem gewünscht hohen Probendurchsatz treffen zu können.

Vor diesem Hintergrund besteht eine technische Aufgabe im Bereitstellen eines Verfahrens und einer entsprechenden Vorrichtung zur objektiven Bewertung von Spannbetonschwellen, insbesondere zur zuverlässigen Bewertung der strukturellen Integrität bereits gebrauchter und einem Gleisbett entnommener Spannbetonschwellen in Hinsicht auf deren Wiederverwendbarkeit in einem Gleisbett.

ZUSAMMENFASSUNG DER ERFINDUNG

Gemäß der vorliegenden Offenbarung wird ein Prüfverfahren und eine Vorrichtung zur automatischen Durchschallung von Spannbetonschwellen mit typischerweise hoher örtlicher Auflösung, insbesondere mit einer Auflösung im Bereich weniger Millimeter oder eines Zentimeters und eine darauf basierende automatische Zustandsbewertung von Spannbetonschwellen vorgeschlagen.

Insbesondere erfolgt die Ankopplung der verwendeten Ultraschallprüfköpfe über eine Vorlaufstrecke, typischerweise eine Wasservorlaufstrecke. Die so durchführbare Durchschallungsprüfung erfolgt durch einen wahlweise kontinuierlich oder diskontinuierlich automatisch vorgenommenen Wechsel der Positionen von paarweise verwendeten Ultraschallprüfköpfen. Typischerweise werden dabei die Prüfköpfe entlang der geprüften Schwelle bewegt. Selbstredend ist es unerheblich, ob hierzu eine Bewegung der Schwelle relativ zu den Prüfköpfen und/oder eine Bewegung der Prüfköpfe relativ zu einer - während der Messung - ortsfest angeordneten Spannbetonschwelle erfolgt. Das vorgeschlagene Verfahren schließt ein automatisches Zuordnen einer Messposition zu einer oder mehreren an dieser Messposition gemessenen Ultraschalllaufzeit(en) ein. Das vorgeschlagene Verfahren der Eignungsprüfung von Spannbetonschwellen umfasst weiterhin die automatisch erfolgende Bewertung der Ultraschalllaufzeit hinsichtlich vorgebbarer Schwellenwerte und damit eine Entscheidung über die Verwendbarkeit der geprüften Spannbetonschwelle, insbesondere über die Wiederverwendbarkeit einer gebrauchten oder wiederaufbereiteten Spannbetonschwelle.

Typische Ausführungsformen und Aspekte der Erfindung ermöglichen es, auf neuartige einfache, vor allem aber zuverlässige Weise, eine automatische Bewertung von Spannbetonschwellen hinsichtlich ihrer Eignung zur bestimmungsgemäßen Verwendung in einem Gleisbett vorzunehmen. Grundlage der automatischen Bewertung ist eine automatisch erfolgende Verschiebung der Position eines oder typischerweise mehrerer Paare von Ultraschallprüfköpfen relativ zur Spannbetonschwelle, d.h. ein automatisch erfolgender Wechsel von Messpositionen bei der zerstörungsfreien Durchschallungsprüfung der Spannbetonschwelle.

KURZBESCHREIBUNG DER FIGUREN

Ausführungsformen der Erfindung sind in den Zeichnungen dargestellt und in der nachfolgenden Beschreibung näher erläutert. In den Zeichnungen zeigen:

**Fig. 1** - die Anordnung eines Prüfkopfpaares für die hier vorgeschlagene Ultraschallprüfung einer Spannbetonschwelle in einem entsprechenden System 100. **Fig. 1A:** Frontalansicht; **Fig. 1B:** Seitenansicht; **Fig. 1C:** Draufsicht.

**Fig. 2** - die Anordnung von mehreren Prüfköpfen (jeweils Sender und Empfänger) bei der hier vorgeschlagenen Prüfung mit mehreren Prüfkopfpaaren, hier drei in einem System 110. **Fig. 2A:** Frontalansicht; **Fig. 2B:** Seitenansicht. Bezugszeichen wie in **Fig. 1.**

**Fig. 3** - ermittelte Werte der Ultraschalllaufzeit einer beispielhaften Ultraschallmessung an einer Spannbetonschwelle mit einem Prüfkopfpaar und deren Lage in Bezug auf einen festgelegten Toleranzbereich.

**Fig. 4** - Bezeichnungen und Funktionsstrukturen von Spannbetonschwellen 1.

**Fig. 5** - schematisch die zur Bestimmung der Ultraschallaufzeit genutzte Messanordnung 120 bzw. das entsprechende System 120 mit den für die Bestimmung der Durchschallungszeit maßgeblichen Längen.

**Fig. 6** - eine schematische Erläuterung zur Orientierung von Beschallungsrichtung und dem Verlauf der resultierenden Durchschallungsachse in der Spannbetonschwelle.

**Fig. 7** - grobschematisch eine Anlage 130 bzw. ein System 130 zur kontinuierlichen (Durchlauf-)Prüfung einer Vielzahl von Spannbetonschwellen 1 mit zugehörigen Transportstrecken 9, 9.1, 9.21, 9.22.

**Fig. 8** - ein Ablaufschema der Schrittfolge des vorgeschlagenen Verfahrens 1000.

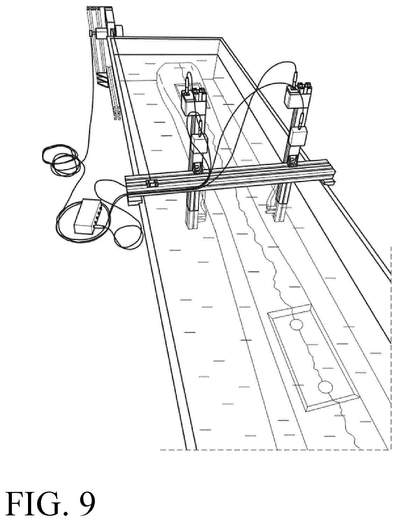

**Fig. 9** - eine zum Nachweis der prinzipiellen Machbarkeit verwendete Messanordnung.

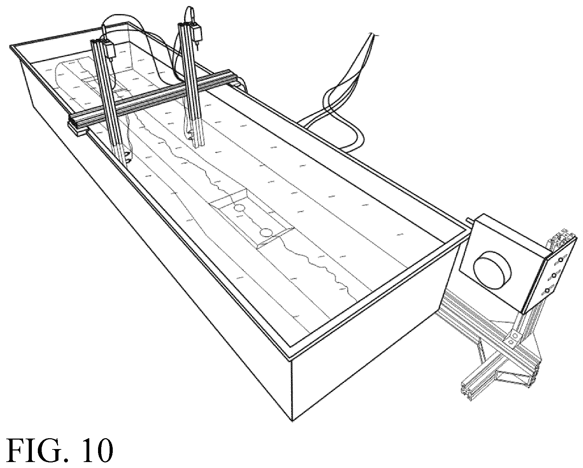

**Fig. 10-** eine andere Ansicht der in Fig. 9 gezeigten Messanordnung.

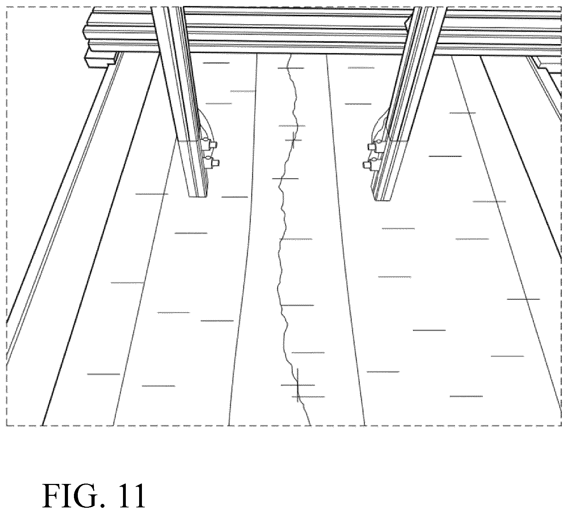

**Fig. 11-** eine Detailansicht einer Anordnung von zwei Paaren Ultraschallprüfköpfe.

**Fig. 12-** ein Schema zu erwartender Durchschallungsstrecken bei der Durchschallung einer Spannbetonschwelle 8 (ohne die Berücksichtigung von Brechungen) in vier übereinander liegenden Ebenen mit vier Paaren Ultraschallprüfköpfen.

**Fig. 13** - ermittelte Werte der Ultraschalllaufzeit einer beispielhaften Ultraschallmessung an einer Spannbetonschwelle mit einem Prüfkopfpaar und deren Lage in Bezug auf einen festgelegten Schwellenwert.

**Fig. 14** - die Orientierung des als Sender konfigurierten Ultraschallprüfkopfs unter einem definierten Winkel zu einer Oberflächennormalen der Oberfläche der Spannbetonschwelle in den Messpositionen gemäß der Gleichung: $\sin \vartheta_a / c_a = \sin \vartheta_b / c_b$ wobei c für die Schallgeschwindigkeit in a - dem Koppelfluid und b - im Beton steht.

GENERELLE ASPEKTE DER ERFINDUNG

Gemäß praxisüblichem Vorgehen werden Spannbetonschwellen nach dem Ausbau aus dem Gleis in Aufbereitungswerken visuell auf Schäden und ihre mögliche Wiederverwertbarkeit geprüft. Dieses manuelle Vorgehen ist aufwendig und fehleranfällig.
Vor diesem Hintergrund wird vorgeschlagen, die Integrität des Betons der Spannbetonschwelle automatisiert mit Ultraschall per Durchschallung zu prüfen. Das ermöglicht es, auch äußerlich nicht sichtbare Schäden zu detektieren bzw. die weitere Eignung der Spannbetonschwellen zuverlässig zu beurteilen und zwar auch dann, wenn die Schwelle keine äußerlich sichtbaren Schäden aufweist. Verfahrensgemäß wird die Spannbetonschwelle für die Prüfung typischerweise in ein Wasserbad gelegt. Das erübrigt eine zusätzliche Reinigung der geprüften Schwelle, um das zur Prüfung verwendete Koppelmittel zu entfernen. Mindestens ein Paar, das heißt mindestens zwei wasserdichte (oder wasserdicht verpackte) Ultraschallprüfköpfe 3 (Sender 3.1 und Empfänger 3.2) werden beidseitig der Schwelle 1 in definiertem Abstand $l_{c1}$ und $l_{c2}$ zur äußeren Oberfläche der Schwelle entlang einer gedachten Durchschallungsachse angeordnet. Die Spannbetonschwelle ist vollständig vom Ankopplungsmedium umschlossen, also vollständig im Wassert oder der wässrigen Lösung untergetaucht. Die Ultraschalllaufzeit zwischen Sender und Empfänger wird gemessen. Selbstredend ist die erreichte Auflösung der Größe (lokale Ausdehnung) der Fehlstellen von der Frequenz der zur Prüfung eingesetzten Ultraschallsignale und der Schallgeschwindigkeit im Beton abhängig. Frequenzen im Bereich von 50 - 500 kHz, bevorzugt im Bereich 100 kHz - 300 kHz, weiter bevorzugt 150 kHz - 200 kHz liefern für den in Schwellen vorliegenden Beton eine Auflösung von 100 mm bis 10 mm; 50 mm bis 17 mm, bzw. 33 mm - 25 mm.

Tabelle 1

| Schallgeschwindigkeit [m/s] | Frequenz [kHz] | Wellenlänge [mm] |
|---|---|---|
| 5000 | 50 | 100 |
| 5000 | 500 | 10 |
| 5000 | 100 | 50 |
| 5000 | 300 | 17 |
| 5000 | 150 | 33 |

Die Auflösung der Größe der Fehlstellen liegt etwa im Bereich der Wellenlänge der Ultraschallwellen, wobei Wellenlänge = Schallgeschwindigkeit / Frequenz (vgl. Tabelle 1).

Erfindungsgemäß wird die gemessene Ultraschalllaufzeit als Maß zur Beurteilung der Gefügeintegrität verwendet. Dabei wird unter einem intakten Gefüge das Fehlen von Rissen im Beton verstanden. Erfasste Risse sind sowohl Makrorisse oder aus Treibreaktionen resultierende und typischerweise über ein größeres Volumen verteilte Mikrorisse, die zu einer Gefügeauflockerung führen. Nachgewiesen werden ebenso große Luftporen und Verdichtungsmängel, z.B. Kiesnester im Beton. Bekannte Ultraschalllaufzeiten ermöglichen Rückschlüsse auf vorhandene Schäden (z.B. (netzartig) verteilte Mikrorisse, Makrorisse, Hohlräume und andere Inhomogenitäten des Betons). Unter Berücksichtigung der Vorlaufstrecke der Schallwellen im Ankopplungsmedium (typischerweise Wasser) kann die Schallgeschwindigkeit im Beton der aktuell geprüften Spannbetonschwelle berechnet werden. Für die an Spannbetonschwellen messbaren Schallgeschwindigkeiten (oder Ultraschalllaufzeiten) können für den betreffenden Beton der aktuell vorliegenden Spannbetonschwelle Grenzwerte, also Schwellenwerte oder sogar Toleranzbereiche festgelegt werden. Ein Überschreiten des betreffenden Schwellenwerts für eine Ultraschalllaufzeit weist dann auf das Vorliegen von Gefügestörungen oder andere kritische Defekte hin. Das vorgeschlagene Prüfverfahren erlaubt es mithin, vorgeschädigte Spannbetonschwellen von einer Wiederverwendung in einem Gleisbett auszuschließen und damit die (Wieder-) Einsetzbarkeit der betreffenden Spannbetonschwelle anzuzeigen. Im Ergebnis kann dadurch, und zwar ohne die Sicherheit des Schienenfahrzeugverkehrs zu beeinträchtigen, die Anzahl neu zu produzierender Spannbetonschwellen reduziert werden, was auch aus ökologischen Gründen wünschenswert ist.

Zur Ermittlung betreffender Schwellenwerte oder für die Festlegung zulässiger Abweichungen, also letztlich von Toleranzbereichen, werden typischerweise fabrikneue, zuvor nicht belastete Spannbetonschwellen der gleichen Bauform vermessen.

Überraschend erwies sich, dass typischerweise ausschließlich im Verfahren Schallemissionsprüfung verwendete Prüftechnik (Ultraschallgeneratoren, Schallemissionsprüfköpfe) als hochauflösendes Ultraschallprüfsystem eingesetzt werden kann. Mess- bzw. Wandlungsprinzip Sensoren und Sender: piezoelektrisch.

Für die hier beschriebenen Untersuchungen wurden Schallemissionssensoren und ein Schallemissionssystem der Firma Vallen (www.vallen.de) mit folgenden Sensoren verwendet:

- Vallen VS150-K3: Frequency Range (fPeak) [kHz] 100 to 450 (150), IP68 bis max. 10 bar.

Alternativ einsetzbar (nicht getestet) sind Sensoren der Typen:

- Vallen VS375-WIC-V01: Frequency Range (fPeak) [kHz] 250 to 700 (375), IP68, max. 60 bar (with connected cable)

- Vallen VS900-WIC-V01: Frequency Range (fPeak) [kHz] 100 to 900 (350), IP68, max. 60 bar (with connected cable)

- Olympus: 500kHz: A391S-SU oder V391-SU oder A389S-SU oder V389S-SU.

Es erwies sich als vorteilhaft, statt eine Ultraschallprüfsystems ein Schallemissionssystem zu verwenden. Das genutzte System AMSY 6 der Firma Vallen ermöglicht es, einen Spannungspuls (150 kHz, max. 450 $V_{PP}$) an einen beliebigen Sensor zu senden und ihn somit als Ultraschallsender zu betreiben. Gleichzeitig können die an allen anderen Sensoren ankommenden Signale synchron aufgezeichnet werden.

Gemäß typischen Ausführungsformen kann das hier beschriebene Verfahren der Ultraschallprüfung von Spannbetonschwellen beispielsweise mit 150-kHz Prüfköpfen vorgenommen werden.

Im Folgenden werden einige weitere Aspekte der Erfindung beschrieben. Sofern nicht ausdrücklich anders angegeben, sind diese Aspekte unabhängig voneinander und können, soweit technisch möglich und sinnvoll, in jeglicher Weise miteinander kombiniert werden. Zum Beispiel kann jeder in dieser Offenbarung beschriebene Aspekt mit jedem anderen Aspekt bzw. jedem hierin beschriebenen Ausführungsbeispiel kombiniert werden, um so weitere Aspekte bzw. Ausführungsbeispiele zu erhalten.

Gemäß einer Ausführungsform wird ein Verfahren zur Prüfung von Spannbetonschwellen auf deren bestimmungsgemäße Verwendbarkeit als Gleisschwelle in einem Gleisbett vorgeschlagen. Dieses Verfahren umfasst:

a. Anordnen der zu prüfenden Spannbetonschwelle in einem Ankopplungsfluid;

b. Positionieren eines Paares von Ultraschallprüfköpfen beidseitig der zu prüfenden Spannbetonschwelle in einem jeweiligen definierten Abstand $l_{c1}$, $l_{c2}$ und in einem jeweilig definierten Winkel zu Oberflächen der Spannbetonschwelle in mindestens einer Messposition;

c. Aussenden eines Ultraschallsignals durch einen als Sender konfigurierten Ultraschallprüfkopf des Paares von Ultraschallprüfköpfen und Empfangen eines Durchschallungssignals durch einen als Empfänger konfigurierten Ultraschallprüfkopf des Paares von Ultraschallprüfköpfen, wobei das Aussenden und das Empfangen in der mindestens einen Messposition erfolgt;

d. Ermitteln einer Ultraschalllaufzeit $t_s$ des in der mindestens einen Messposition ausgesendeten und empfangenen Ultraschallsignals im Beton der Spannbetonschwelle;

e. Bewerten der Spannbetonschwelle als tauglich für die Verwendung als Gleisschwelle im Gleisbett, wenn die ermittelte Ultraschalllaufzeit in jeder Messposition der mindestens einen Messposition unterhalb eines jeweiligen Schwellenwertes liegt oder dem jeweiligen Schwellenwert entspricht, und als untauglich für die Verwendung der Spannbetonschwelle als Gleisschwelle im Gleisbett, wenn die ermittelte Ultraschalllaufzeit in wenigstens einer Messposition der mindestens einen Messposition oberhalb des jeweiligen Schwellenwertes liegt.

Selbstredend können auch andere, mit der Ultraschalllaufzeit verknüpfte oder von dieser ableitbare Größen zur Festlegung eines Schwellenwertes oder eines Toleranzbereiches zur Bewertung der aktuell geprüften Spannbetonschwelle genutzt werden. Vorteilhaft an dem vorgeschlagenen Verfahren ist der durch das vergleichsweise simple Messprinzip erreichbare hohe Probendurchsatz. Das heißt, dass allein an Hand von aufeinander abgestimmten Durchschallungspulsen eine Spannbetonschwelle über ihre volle Länge in vergleichsweise kurzer Zeit geprüft werden kann. Das Verfahren ermöglicht es, bei einer entsprechenden Anordnung der Paare von Ultraschallprüfköpfen, beispielsweise versetzt übereinander in unterschiedlichen Ebenen, die Spannbetonschwelle wenn gewünscht auch detaillierter an besonders kritischen Abschnitten oder als besonders kritisch und damit für eine Wiederverwendbarkeit als relevant erachteten Bereichen zu prüfen.

Gemäß einer Ausführungsform ist im vorstehend vorgeschlagenen Verfahren das Ankopplungsfluid im Schritt Anordnen (a.) ausgewählt unter einer Flüssigkeit, insbesondere unter einer wässrigen Lösung und Wasser.

Vorteilhaft kann so eine gegebenenfalls zuvor einer Hochdruckreinigung mit Wasser, eventuell sogar unter Zusatz eines Tensids, unterzogene Spannbetonschwelle direkt in das artgleiche Kopplungsfluid überführt werden. Auch aus der Sicht der Umweltverträglichkeit ist die Verwendung von Wasser oder wässrigen Lösungen der Nutzung höherviskoser Medien vorzuziehen. Gegenüber Luft oder anderen Fluiden gewährleisten wässrige Lösungen und Wasser eine effektive Signalführung (Ein- bzw. Auskopplung).

Gemäß einem Ausführungsbeispiel erfolgt das Ermitteln der Ultraschalllaufzeit (Schritt d) aus einer Differenz eines Zeitpunktes des Aussendens des Ultraschallsignals und eines Zeitpunktes des Empfangens des Durchschallungssignals unter Berücksichtigung einer Länge einer Ankopplungsfluid-Durchschallungsstrecke $l_{c1}$, $l_{c2}$ im verwendeten Ankopplungsfluid und einer Länge einer Beton-Durchschallungsstrecke $l_s$ im Beton der Spannbetonschwelle.

Vorteilhaft ist die Ultraschalllaufzeit als primäre Messgröße vergleichsweise einfach und störungsfrei exakt zu ermitteln.

Gemäß einer Ausführungsform umfasst das vorgeschlagene Verfahren weiter ein Anordnen des Paares Ultraschallprüfköpfe in der mindestens einen Messposition (Schritt f) mit Bezug zu einem markanten Bezugspunkt der Spannbetonschwelle und/oder - wenn der aktuell vorzunehmenden Messung bereits eine Messung vorausging - Verschieben des Paares Ultraschallprüfköpfe (Schritt g) aus einer Messposition der mindestens einen Messposition relativ zu der Lage des markanten Bezugspunktes der zu prüfenden Spannbetonschwelle oder einer Bezugsgeraden der zu prüfenden Spannbetonschwelle in eine weitere Messposition der mindestens einen Messposition.

Vorteilhaft können, sollte beispielsweise eine Vielzahl von Ultraschallprüfköpfen eingesetzt werden, allein wenige derartige Verschiebungsschritte oder sogar nur ein einmaliges Verschieben eine vollständige Durchschallung und damit Prüfung der Spannbetonschwelle ermöglichen. Dadurch kann ein erreichbarer Prüfdurchsatz erheblich gesteigert werden.

Gemäß einer Ausführungsform umfasst das Anordnen und/oder Verschieben gemäß Schritt f. bzw. Schritt g. ein automatisches Bewegen der Spannbetonschwelle (1) aus einer vorausgehenden Position der Spannbetonschwelle, wobei das automatische Bewegen der Spannbetonschwelle insbesondere kontinuierlich oder diskontinuierlich erfolgt.

Vorteilhaft ermöglicht das eine präzise Anordnung der Spannbetonschwelle relativ zu dem zumindest einen Paar Ultraschallprüfköpfe.

Gemäß einer Ausführungsform erfolgt das automatische Bewegen der Spannbetonschwelle diskontinuierlich und das Aussenden und das Empfangen von Ultraschallsignalen erfolgt während eines Verweilens bzw. Verharrens der Spannbetonschwelle in einer jeweils eingenommenen Messposition.

Vorteilhaft kann über vergleichsweise einfache mechanische Vorrichtungen eine ruckartige Vorwärtsbewegung der Spannbetonschwelle unter Einhaltung einer konstanten "Schrittweite" gewährleistet werden. Die relativ große Masse der Spannbetonschwelle gewährleistet eine stabile Lage der Spannbetonschwelle während der Messung, während ein entsprechend gewählter Pegelstand des Ankopplungsfluids und/oder eventuell vorgesehene "Wellenbrecher" ein vollständiges Benetzen bzw. Umschließen der Betonschwelle durch das Ankopplungsfluid gewährleisten. Damit ist die Zuverlässigkeit der jeweils erhobenen Ultraschalllaufzeiten gesichert. Selbstredend kann sowohl die Spannbetonschwelle im Prüfbecken entlang der Anordnung der Ultraschallprüfköpfe bewegt werden, ebenso aber kann auch die Anordnung der Ultraschallprüfköpfe entlang der Spannbetonschwelle bewegt werden. Die letztgenannte Variante dieser Ausführungsform erscheint vorteilhaft für ein automatisch und über die gesamte Länge der Betonschwelle gleichmäßig erfolgendes Ablegen der Spannbetonschwelle in einem mit dem Ankopplungsmedium gefüllten Prüfbehälter (einer "Wanne"). Das Bewegen der paarweise angeordneten Ultraschallprüfköpfe längs der Spannbetonschwelle ist vorteilhaft unter weitestgehender Vermeidung von störenden Pegelschwankungen (Wellenschlag des Ankopplungsfluids im Prüfbehälter), die den Empfang der Messsignale nachteilig beeinflussen.

Gemäß einer Ausführungsform erfolgt das automatische Bewegen bzw. Verschieben der Spannbetonschwelle kontinuierlich und das Aussenden und das Empfangen von Ultraschallsignalen gemäß Schritt c. erfolgt in einem so bemessenen Zeitraum, dass die aktuelle Messung trotz der kontinuierlichen Bewegung der Spannbetonschwelle einer jeweils eingenommenen Messposition und damit die ermittelten Messergebnisse einem konkreten Abschnitt der Spannbetonschwelle zuordenbar sind.

Vorteilhaft ist diese Maßnahme geeignet, den Durchsatz der geprüften Spannbetonschwellen weiter zu steigern, ohne die Zuverlässigkeit der erhobenen Befunde "Geeignet / Ungeeignet" zu vermindern.

Gemäß einer Ausführungsform erfolgt das kontinuierliche automatische Bewegen bzw. das kontinuierliche automatische Verschieben mit einer Geschwindigkeit von 0.5 cm/s bis 2,5 cm/s, insbesondere mit einer Geschwindigkeit von 1 cm/s, wobei eine Pulsfolgefrequenz kleiner als 20 Hz, insbesondere ≤ 12 Hz, bevorzugt ≤ 10 Hz ist.

Die bezeichnete Kombination von Geschwindigkeit und Pulsfolgefrequenz ermöglicht störungsfreie Messungen der Ultraschalllaufzeiten an zueinander nahen Abschnitten der Spannbetonschwelle.

Gemäß einer Ausführungsform sind die, bevorzugt paarweise verwendeten, Ultraschall-Prüfköpfe für einen Frequenzbereich von 30 kHz bis 1000 kHz, bevorzugt für einen Bereich von 100 kHz bis 500 kHz, weiter bevorzugt für einen Bereich zwischen 100 kHz bis 200 KHz ausgelegt.

Vorteilhaft erwies sich, dass die für eine Signalerfassung in diesem Frequenzbereich ausgelegte Ultraschallprüfköpfe sich ausgezeichnet auch für die Erzeugung von Ultraschallsignalen ausreichend hoher Intensität (Amplitude) eignen.

Gemäß einer Ausführungsform übersteigt eine als Pulsfolgefrequenz definierte Abfolge von aufeinanderfolgend ausgesendeten Ultraschallpulsen einen Wert von 20 Hz nicht. Insbesondere ist die Pulsfolgefrequenz ≤ 10 Hz.

Vorteilhaft ermöglicht das störungsfreie Messungen der Ultraschalllaufzeiten selbst an unmittelbar zueinander benachbarten Abschnitten der Spannbetonschwelle.

Gemäß einer Ausführungsform umfasst das vorgeschlagene Prüfverfahren ein Definieren (bzw. Festlegen) der mindestens einen Messposition als innerhalb eines Bereichs der Einleitung einer Vorspannkraft in den Beton der Spannbetonschwelle (1) liegend. Dabei kann dieses Definieren auch unabhängig vom jeweiligen Typ der Spannbetonschwelle oder deren Hersteller erfolgen, beispielsweise sowohl für Spannbetonschwellen des Typs B70 als auch des Typs B90.

Vorteilhaft ist die anhand einer herstellerseitigen Markierung exakt bekannte Konstruktion der Spannbetonschwelle geeignet, den Bereich der Spannbetonschwelle zu identifizieren, an dem die Vorspannung der Armierungsstähle in den Beton eingeleitet wird. Gerade die Kenntnis des Gefügezustandes dieser Abschnitte der Betonschwelle ist für die Beurteilung einer zuvor gebrauchten Spannbetonschwelle auf ihre Wiederverwendbarkeit in einem Gleisbett entscheidend.

Gemäß einer Ausführungsform ist die Spannbetonschwelle eine zuvor gebrauchte und einem Gleisbett entnommene Spannbetonschwelle.

Spannbetonschwellen sind über ihre Nutzungsdauer verschiedenen Umwelteinflüssen und Lasteinträgen ausgesetzt. Daraus kann sich, beispielsweise bedingt durch eine aggregatverursachte Alkali-Kieselsäure-Reaktion im Beton eine fortwährende Degradation des Gefüges bis zu einer vollständigen Zerrüttung ergeben. Hieraus ergibt sich das Erfordernis, teilweise ganze Streckenabschnitte mit neuen Gleisen zu versehen. Vor diesem Hintergrund ist die Unterscheidung von für eine Wiederverwendung geeigneten Spannbetonschwellen von geschädigten Spannbetonschwellen sinnvoll. Die Autoren der vorliegenden Schrift haben herausgefunden, dass sich die Degradation des Gefüges bzw. die mechanische Stabilität der Spannbetonschwelle über Messung der Schalllaufzeit (von L), deren Quadrat für Longitudinalwellen proportional zum Quotient aus Dichte und Elastizitätsmodul ist, in den jeweiligen Messpositionen mit bekannter Schallstrecke zuverlässig charakterisieren lässt. Eine explizite (bildbasierte) Charakterisierung einzelner Risse oder anderer Materialfehler ist dabei nicht erforderlich. Dies vereinfacht die Charakterisierung von Spannbetonschwellen hinsichtlich ihrer Verwendbarkeit als Gleisschwelle in einem Gleisbett erheblich.

Gemäß einer Ausführungsform liegt der jeweils definierte Abstand $l_{c1}$, $l_{c2}$ gemäß Schritt b. des vorgeschlagenen Verfahrens in einem Bereich von 0,5 cm bis 10 cm, bevorzugt in einem Bereich von 0,5 cm bis 1 cm.

Vorteilhaft ermöglicht das eine weitestgehend verlustarme Ein- und Auskopplung des Ultraschallprüfsignals und damit eine vorteilhaft hohe Auflösung der Messungen.

Gemäß einer Ausführungsform umfasst das Positionieren des Paares von Ultraschallprüfköpfen gemäß Schritt b. des vorgeschlagenen Verfahrens ein einer äußeren Kontur der Spannbetonschwelle (1) folgendes Nachführen zumindest des als Empfänger konfigurierten Ultraschallprüfkopfes des Paares von Ultraschallprüfköpfen.

Das erhöht die Präzision des Nachweises von Gefügestörungen.

Gemäß einer Ausführungsform wird der gemäß Schritt b. des vorgeschlagenen Verfahrens jeweils definierte Winkel ausgedrückt als Winkel zu einer Oberflächennormalen der Oberfläche der Spannbetonschwelle in den Messpositionen gemäß der Gleichung:

$$\sin \vartheta_a / c_a = \sin \vartheta_b / c_b$$

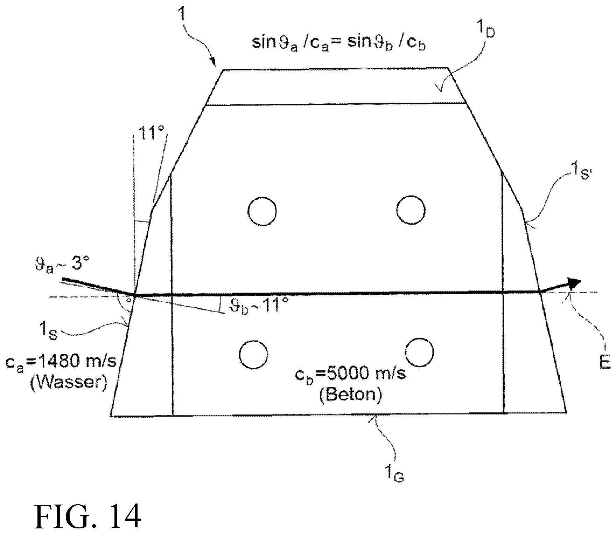

Dabei beschreibt $c_b$ die Schallgeschwindigkeit im Beton der Spannbetonschwelle (1), $c_a$ die Schallgeschwindigkeit des Ankopplungsfluids, und $\vartheta_b$ einen Winkel der Oberflächennormalen der Oberfläche (Seitenfläche in Fig. 14) $1_S$ (bzw. der Seitenfläche $1_{S'}$) der Spannbetonschwelle (1) bezüglich einer Grundfläche $1_G$ der Spannbetonschwelle 1, einer Deckfläche $1_D$ der Spannbetonschwelle 1 und/oder einer Ebene E, in der das durch den als Sender konfigurierten Ultraschallprüfkopf des Paares von Ultraschallprüfköpfen ausgesandte Ultraschallsignal (roter Pfeil in Fig. 14) die Spannbetonschwelle 1 in der jeweiligen Messposition die Spannbetonschwelle 1 durchlaufen soll, wobei die Indices a für das Koppelmedium und b für Beton steht und/oder der Winkel bei etwa 3° liegt.

Vorteilhaft ist so eine primär zur Grundfläche der Spannbetonschwelle parallel verlaufende Ausbreitungsrichtung des Ultraschallsignals in der Spannbetonschwelle erreichbar.

Gemäß einer Ausführungsform wird der jeweils definierte Winkel gemäß Schritt b. des vorgeschlagenen Verfahrens so gewählt ist, dass eine Ausbreitungsrichtung einer durch das Aussenden des Ultraschallsignals im Beton der Spannbetonschwelle generierten Transversalwelle mit einer im Beton der Spannbetonschwelle (1) generierten Longitudinalwelle weitestgehend zusammenfallen.

Vorteilhaft wird der jeweils definierte Winkel gemäß Schritt b. des vorgeschlagenen Verfahrens so gewählt ist, dass eine Ausbreitungsrichtung einer im Beton der Spannbetonschwelle generierten Longitudinalwelle weitestgehend parallel zur Grundfläche der Spannbetonschwelle verläuft.

Gemäß einer Ausführungsform umfasst das das Anordnen gemäß Schritt a. des vorgeschlagenen Verfahrens die Schritte:

a1. Entnehmen der Spannbetonschwelle aus dem Gleisbett;

a2. Reinigen der entnommenen Spannbetonschwelle von oberflächlichen Verunreinigungen, und/oder

a3. Überführen der gereinigten Spannbetonschwelle in einen Prüfbehälter, der mit dem Ankopplungsfluid gefüllt ist, wobei ein Pegelstand des Ankopplungsfluids < 0,5 mal höher ist, als die am Grunde des Prüfbehälters angeordnete Spannbetonschwelle.

Vorteilhaft ermöglicht das ein störungsfreies Vermessen der Ultraschalllaufzeiten in den Spannbetonschwellen.

Gemäß einer Ausführungsform umfasst das vorgeschlagene Verfahren weiterhin ein Bestimmen des jeweiligen Schwellenwertes für die Ultraschalllaufzeit in der mindestens einen Messposition mittels einer Vergleichsmessung an einer Vergleichsspannbetonschwelle. Als Vergleichsbetonschwelle kommen bevorzugt fabrikneue Spannbetonschwellen desselben Typs und desselben Herstellers in Betracht. Um das vorgeschlagene Verfahren zu verifizieren, wurden Vergleichsmessungen an mehreren mit anderen Verfahren als ungeeignet klassifizierten Spannbetonschwellen vorgenommen und dabei die Eignung und Treffsicherheit des hier vorgeschlagenen Verfahrens bestätigt. Ebenso ist die Ermittlung von Vergleichswerten an Hand einer numerischen Simulation der Schallausbreitung in einem Modell für die Spannbetonschwelle möglich.

Gemäß einer Ausführungsform wird ein System zur Prüfung und/oder zur Zertifizierung eines Herstellungsverfahrens einer Spannbetonschwelle oder zur Zertifizierung einer Spannbetonschwelle für deren Verwendbarkeit in einem Gleisbett vorgeschlagen. Das vorgeschlagene System umfasst:

- zumindest ein Paar Ultraschallprüfköpfe;

- ein Ankopplungsmedium bzw. ein Ankopplungsfluid, beispielsweise eine wässrige Lösung oder Wasser;

- ein Bewegungsmittel, ausgewählt unter einem Prüfkopfbewegungsmittel und einem Spannbetonschwellenbewegungsmittel;

- eine Kontroll- und Steuereinheit, die konfiguriert ist, das zumindest eine Paar Ultraschallprüfköpfe und das Bewegungsmittel so anzusteuern, dass das System ein Verfahren nach einem der vorhergehenden Ansprüche ausführt.

Sich aus dem Betrieb bzw. der Verwendung des vorgeschlagenen Systems ergebenden Vorteile sind die vorstehend genannten.

Gemäß einer Ausführungsform liegt ein Abstand zwischen in Längsrichtung der Spannbetonschwelle zur Ermittlung der Folge von Ultraschalllaufzeiten eingenommenen jeweiligen Messpositionen in einem Bereich von 1 - 100 mm, bevorzugt in dem Bereich von 2 bis 10 mm.

Vorteilhaft erlauben derart geringe Abstände die zuverlässige Erfassung von Ultraschalllaufzeiten in verschiedenen Abschnitten der geprüften Spannbetonschwelle.

Gemäß einer Ausführungsform ist das das Bewegungsmittel des Systems eingerichtet zur reproduzierbaren Positionierung des Paares Ultraschall-Prüfköpfe an einer Vielzahl von Messpositionen, die in einer Ebene liegen, die orthogonal zu einer Durchschallungsachse ausgerichtet ist. Hierbei ist die die Durchschallungsachse parallel einer Längsachse der Spannbetonschwelle oder orthogonal zu einer Ebene, die durch die Längsachse der Spannbetonschwelle (1) definiert wird, orientiert.

Dabei wird besondere Aufmerksamkeit auf die reproduzierbare Einstellung eines Neigungswinkels der zentralen Hauptachse des als Ultraschallsender eingerichteten Ultraschallprüfkopfes des zumindest einen Paares von Ultraschallprüfköpfen gegenüber einer Oberflächennormalen der jeweiligen Oberfläche der Spannbetonschwelle gerichtet.

Hieraus ergeben sich eindeutig und reproduzierbar für verschiedene Spannbetonschwellen bestimmbare Ultraschalllaufzeiten. Aus dem Vergleich der typischerweise an einer Vielzahl von fabrikneuen Spannbetonschwellen definierten Schwellenwerte mit den aktuell ermittelten Ultraschalllaufzeiten ergeben sich so zuverlässige Aussagen über eine Eignung der aktuell geprüften Spannbetonschwelle zur Möglichkeit einer Wiederverwendung in einem Gleisbett.

Gemäß einer Ausführungsform wird die Verwendung einer Messanordnung zur Bestimmung einer Ultraschalllaufzeit in einem Festkörper für die Prüfung einer Spannbetonschwelle auf deren bestimmungsgemäße Verwendbarkeit als Gleisschwelle in einem Gleisbett vorgeschlagen, wobei die Messanordnung ein System gemäß der vorstehend beschriebenen Ausführungsformen umfasst.

Resultierende Vorteile wurden zuvor bereits beschrieben.

Gemäß einer Ausführungsform ist mindestens ein weiteres Paar Ultraschallprüfköpfe so angeordnet, dass die Signale der verschiedenen Prüfkopfpaare sich während einer Messung oder von unmittelbar aufeinander folgenden Messungen nicht gegenseitig beeinflussen und somit einerseits eine gleichzeitige Prüfung der Spannbetonschwelle in verschiedenen Messebenen und also in verschiedenen Messpositionen und/oder eine zeitsparende Prüfung ermöglicht wird.

Sich ergebende Vorteile für eine hohe Reproduzierbarkeit der Messergebnisse, Zuverlässigkeit der Aussagen über den Gefügezustand des Betons im geprüften Abschnitt der Spannbetonschwelle, den Durchsatz an geprüften Spannbetonschwellen je Messplatz, sowie letztlich die Treffsicherheit der resultierenden Entscheidung über die Wiederverwendbarkeit der Spannbetonschwelle sind eingedenk der verschiedenen, oben angegebenen Vorteile einzelner Ausführungsformen offensichtlich.

Gemäß einer Ausführungsform wird die Verwendung einer nach einem Verfahren gemäß einem der Ausführungsformen des vorgeschlagenen Verfahrens als tauglich bewerteten Spannbetonschwelle als Gleisschwelle in einem Gleisbett vorgeschlagen.

Die vorstehend beschriebenen Ausführungsformen können frei miteinander kombiniert werden.

AUSFÜHRLICHE BESCHREIBUNG DER ERFINDUNG

Nachstehend werden Ausführungsformen der Erfindung näher erläutert. Die Zeichnungen dienen der Veranschaulichung eines oder mehrerer Beispiele möglicher Ausführungsformen der Erfindung.

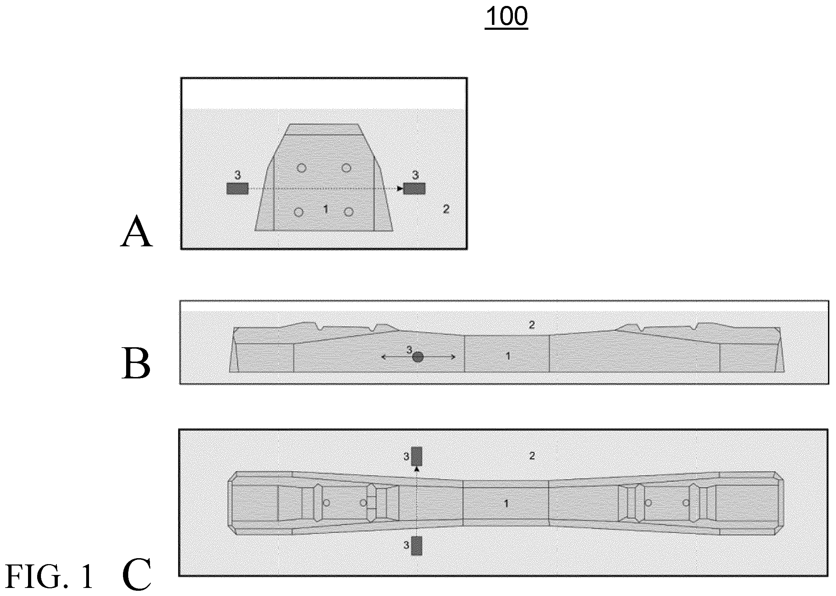

Wie in Fig. 1 dargestellt, können die zur Ultraschallprüfung verwendeten Ultraschallprüfköpfe (Sender und Empfänger) jeweils paarweise, so unter einem optimalen Winkel gegenüber einer Flächennormalen der jeweils beschallten Oberfläche geneigt, an einer Geraden ausgerichtet werden, sodass eine Durchschallungsachse bevorzugt orthogonal zu einer zentralen Längsachse der Spannbetonschwelle verläuft. In Fig. 1 steht das Bezugszeichen 1 für die Spannbetonschwelle; das Bezugszeichen 2 für das umgebende Koppelmedium, z.B. Wasser; das Bezugszeichen 3 für die Ultraschallprüfköpfe (Sender bzw. Empfänger).

Dabei geht es im Wesentlichen um die Detektion von Rissen bzw. einer Gefügeauflockerung durch verteilte Mikrorisse infolge einer Alkali-Kieselsäure-Reaktion (bzw. einer allgemeinen Treibreaktion im Beton). Ebenso können Produktionsfehler (z.B. Kiesnester - d.h. eine Anhäufung von Gesteinskörnung ohne Bindemittel) oder Betonabplatzung infolge des Ausbaus bzw. Transports der Spannbetonschwellen, die natürlich auch bei der visuellen Inspektion auffindbar sind, dokumentiert werden.

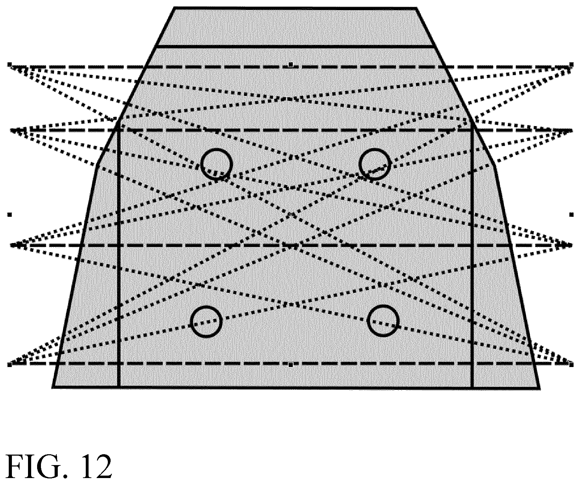

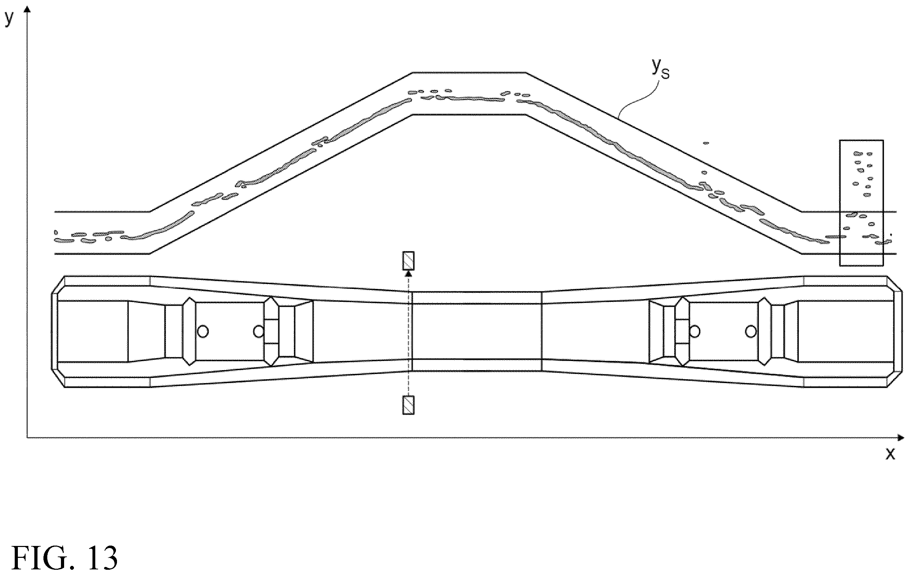

Zwecks eines eineindeutigen Nachweises der vorstehend beschriebenen Schädigungen erfolgt deshalb eine bevorzugte Anordnung der Prüfköpfe in drei bis vier Ebenen, und zwar unter dem unteren Spannstahlpaar; mittig zwischen den Spannstählen; direkt über dem oberen Spannstahlpaar; und ca. 3 cm (ca. 1 Wellenlänge bei 150 kHz) unter der Oberfläche der Schienenauflager (vgl. **Fign. 12, 13**).

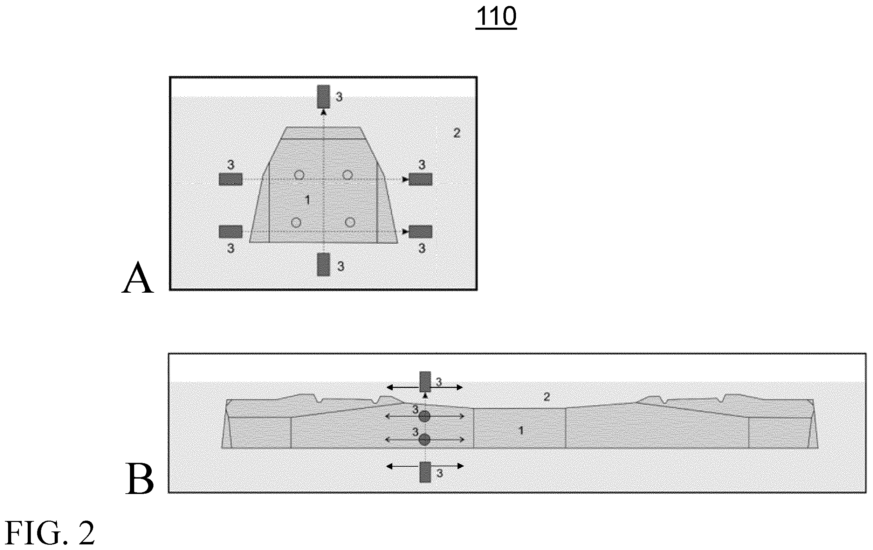

In **Fig. 2** ist beispielhaft die Anordnung von drei Paaren Ultraschallprüfköpfen (jeweils Sender bzw. Empfänger) bei der hier vorgeschlagenen Prüfung mit mehreren Prüfkopfpaaren gezeigt. **Fig. 2A** stellt eine schematische Frontalansicht und **Fig. 2B** eine schematische Seitenansicht (ohne den jeweils optimal eingestellten Neigungswinkel der Ultraschallprüfköpfe zu einer jeweiligen Oberflächennormalen) dar. Die verwendeten Bezugszeichen sind identisch mit den in **Fig. 1** verwendeten.

Die gemäß dieses Schemas vorgeschlagene Durchschallungsprüfung mit drei Paaren Ultraschallprüfköpfen kann beispielsweise so erfolgen, dass die drei Paare starr an einem Rahmen, beispielsweise einem oder zwei U-förmigen Metallrahmen, befestigt werden. Der im Querschnitt beispielsweise umgekehrt U-förmige oder U-förmige Rahmen mit den daran befestigten und jeweils aufeinander ausgerichteten Ultraschallprüfköpfen kann von einem Prüfkopfbewegungsmittel entlang der zu untersuchenden Spannbetonschwelle bewegt werden. Das Bewegungsmittel, beispielsweise ein Kugelwindetrieb, ein Spindelantrieb, ein Schneckengetriebemotor, ein Linearmotor, ein Schraubengetriebe, ein Zahnriemenantrieb, oder ein einfacher Seilzug ist angepasst, eine kontinuierliche oder eine diskontinuierliche Bewegung eines oder mehrerer Prüfkopfpaare unter Einhaltung eines definierten Abstandes zur Schwelle zu gewährleisten.

Beispielsweise kann ein Abstand zur Oberfläche der Spannbetonschwelle konstant gehalten werden. Das impliziert, dass der Weg des durchschallten Kopplungsmediums (typischerweise Wasser) immer konstant ist. Selbstredend kann auch die Spannbetonschwelle an der Anordnung der Prüfkopfpaare vorbeibewegt werden. Ebenso kann in aufeinander folgenden Messungen die Lage der Prüfkopfpaare entlang einer parallel zur zentralen Längsachse der Spannbetonschwelle verlaufenden Geraden verschoben sein. Das ist beispielsweise der Fall, wenn die Prüfkopfpaare wie eingangs erwähnt starr an einem starren U-förmigen Rahmen befestigt sind. Für Paare herkömmlicher Ultraschallprüfköpfe kann bevorzugt eine kontinuierliche Bewegung mit kontinuierlicher Durchschallung der Spannbetonschwelle erfolgen. Die Ultraschallsignale werden dabei gepulst abgegeben, wobei zeitliche Abstände zwischen den Pulsen so gewählt sind, dass keine Beeinflussung der als Empfänger konfigurierten Ultraschallprüfköpfe durch die ihnen benachbarten als Sender konfigurierten Ultraschallprüfköpfe eines benachbarten Prüfkopfpaares erfolgt. Weiterhin sind die zeitlichen Abstände zwischen den Pulsen so einzustellen, dass das zu messende Signal nicht durch den nachfolgenden Puls der nächsten Messposition beeinflusst oder gestört wird.

Gemäß einer Ausführungsform können die Prüfköpfe eines Prüfkopfpaares unabhängig voneinander durch zwei Manipulatoren automatisch an jeweils geeignete Prüfpositionen bewegt werden.

Bei der Verwendung eines Prüfkopfarrays, zumindest auf der Empfängerseite, können auch die Schallpfade zwischen einander nicht direkt gegenüberliegenden Prüfköpfen ausgewertet werden. Verwendet man, wie oben in [0073] beschrieben, Prüfkopfpaare und verwendet jeden Prüfkopf zumindest einer Seite auch als Sender, so können die Spannbetonschwellen pro Messebene auf 16 verschiedenen Pfaden durchschallt werden (vgl. **Fig. 12,** ohne Berücksichtigung der Brechungswinkel).

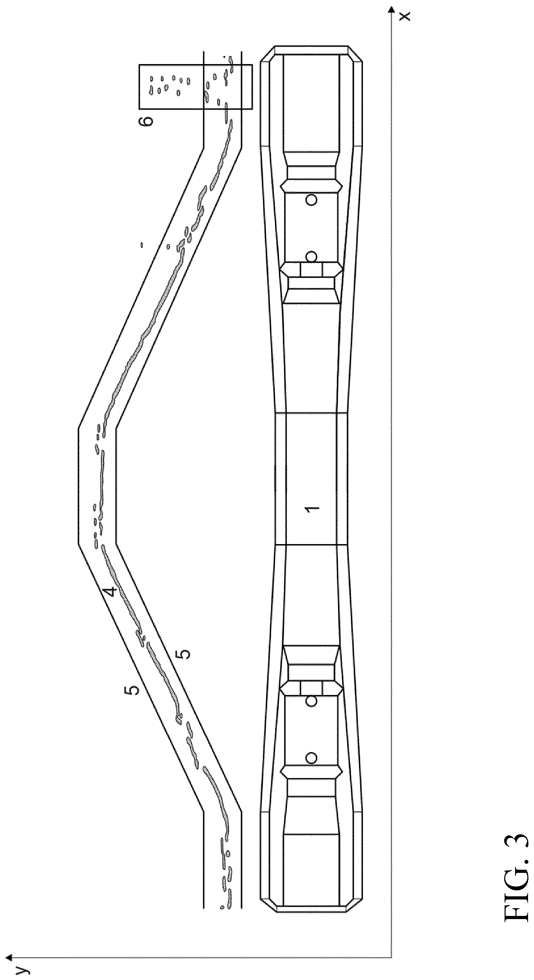

Die **Fig. 3** (und **Fig. 13**) zeigt Messwerte von Ultraschalllaufzeiten, die an einer Spannbetonschwelle mit einem Paar Ultraschallprüfköpfe (3, 3.1, 3.2) bei dessen kontinuierlicher Verschiebung entlang der Spannbetonschwelle ermittelt wurden. Es ist ersichtlich, dass die Prüfköpfe hier entlang einer Geraden bewegt wurden, der Abstand zu Oberflächen der Spannbetonschwelle also gemäß dem Verlauf von deren äußerer Kontur bzw. Geometrie variierte. Die verwendeten Bezugszeichen stehen für: 1 - Spannbetonschwelle; 4 - jeweilige Messwerte der Ultraschalllaufzeit; 5 - tolerierter Bereich für die Ultraschalllaufzeit einer intakten Spannbetonschwelle; 6 - Messwertebereich, der eine Gefüge-Schädigung anzeigt. Insbesondere verdeutlicht die Abszisse (x) die jeweilige Position des Prüfkopfpaares entlang der Spannbetonschwelle, die Ordinate (y-Werte der Messpunkte) entspricht den gemessen Ultraschallaufzeiten (relative Einheiten). Der Verlauf der auf der y-Achse abgetragenen Ultraschalllaufzeit ergibt sich aus dem gleichbleibenden Abstand der Ultraschallmessköpfe zueinander, den unterschiedlichen Schallgeschwindigkeiten in Beton und Wasser und der sich ändernden Geometrie (Dicke) der Spannbetonschwelle 1 (als Funktion von x). Im dargestellten Fall liegen an dem im Bild rechts angeordneten Kopf 14 der Schwelle 1 Gefüge-Störungen vor (vgl. Messwerte "6"), die eine Weiterverwendung der Schwelle 1 als nicht ratsam erscheinen lassen. Die mit dem Bezugzeichen 5 markierten Werte unter- und oberhalb der aktuellen Messwerte 4, tragen der mehr oder weniger heterogenen Zusammensetzung des Betons der Schwelle 1 Rechnung und kennzeichnen einen als Toleranzbereich 5 der Ultraschalllaufzeit festgelegten Bereich. Naturgemäß sind selbst für einen hinsichtlich seiner Zusammensetzung "homogenen" Beton auf Grund der typischerweise vorliegenden Korngrößenverteilung der im Beton zusätzlich zum Zement verwendeten Gesteinskörnung gewisse Abweichungen von einer mittleren Ultraschalllaufzeit für eine identische Durchschallungsstrecke zu erwarten. Liegen aktuell gemessene Werte innerhalb des definierten Toleranz-Bereichs, ist von einer störungsfreien Gefügestruktur auszugehen und die aktuell vermessene Spannbetonschwelle wird als geeignet für eine Wiederverwendung im Gleisbett eingestuft. Typischerweise kann die Prüfung von Spannbetonschwellen 1 auf eine Verwendbarkeit als Gleisschwelle auch nur über den in **Fig. 13** (ortsabhängigen) Schwellenwert $y_s$ (x) für die Ultraschalllaufzeit y erfolgen.

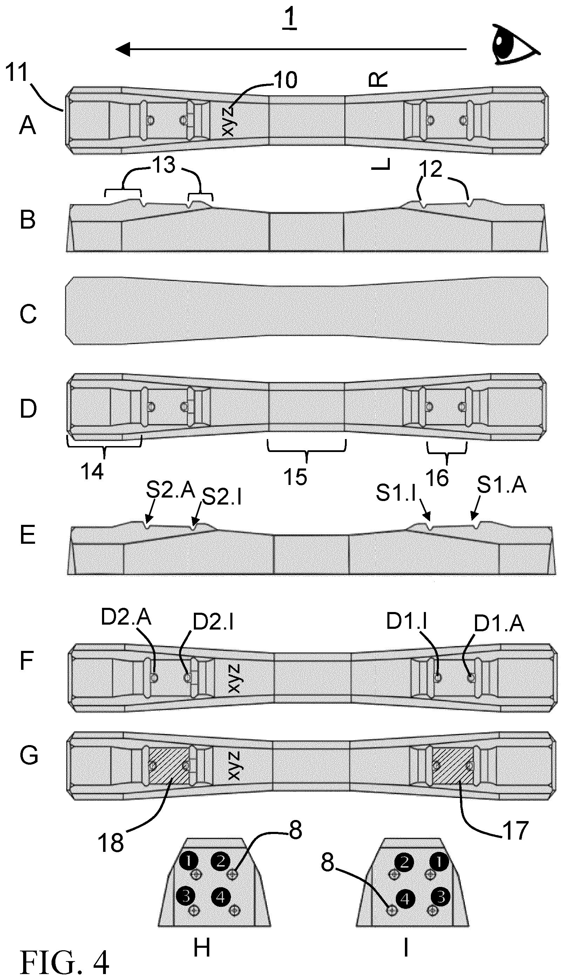

In **Fig. 4** sind funktionale Strukturen und Areale einer typischen Spannbetonschwelle 1 gekennzeichnet. Dabei zeigt der im oberen Bildteil angeordnete Pfeil die zur Orientierung genutzte Blickrichtung. Dabei bezeichnet "R" die in Blickrichtung rechts liegende Seite und "L" die in Blickrichtung links liegende Seite der Spannbetonschwelle 1. A bezeichnet die Aufsicht auf eine Spannbetonschwelle 1. Auf der linken Seite unter der kleinen Sickenschulter 13 trägt die Spannbetonschwelle üblicherweise eine das Herstellerwerk identifizierende Gravur 10. Am linken Kopf 14 der Schwelle 1 trägt die Stirnfläche ggf. eine Datumskappe 11. B zeigt eine Seitenansicht der Spannbetonschwelle bzw. deren Flanke. C zeigt die die Unterseite, D die Oberseite mit Kopf 14, Schwellenmitte 15 und Schienenauflager 16. E bezeichnet nochmals eine Seitenansicht (linke Flanke) mit den vier Sicken 12. Die Sicken 12 umgeben die Schienenauflager 17 und 18, welche ihrerseits unterschieden werden nach ihrer Lage bezogen auf die Blickrichtung und "innen" oder "außen" als S1.A (Sicke 1 außen, S1.I (Sicke 1 innen), S2.I (Sicke 2 innen) und S2.A (Sicke 2 außen). Die Draufsicht F zeigt die analog bezeichneten Dübel 1 und 2, jeweils innen (I) und außen (A) liegend. In Ansicht G ist mit dem Bezugszeichen 17 das erste Schienenauflager und mit dem Bezugszeichen 18 das zweite Schienenauflager bezeichnet. Die beiden Stirnflächen H und I zeigen H: die Anordnung der mit dem Bezugszeichen 8 bezeichneten Spanndrähte 1-4 in Blickrichtung Schild, und I: die Anordnung der Spanndrähte 1-4 in entgegengesetzter Blickrichtung.

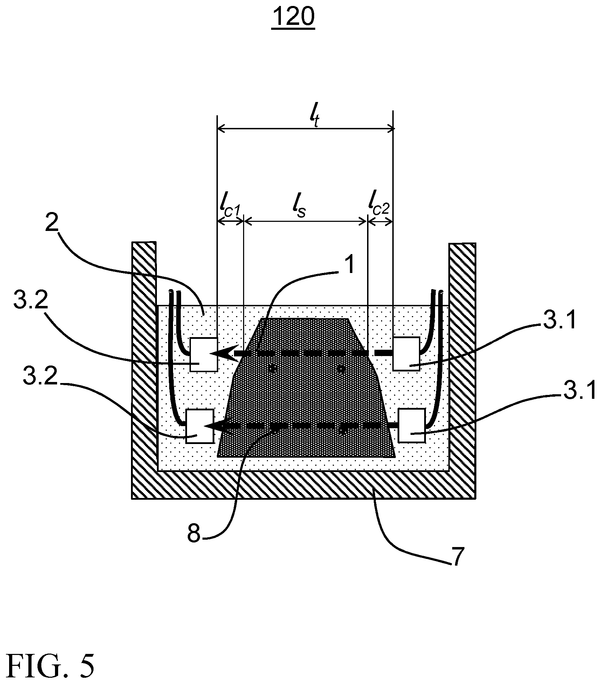

In **Fig. 5** ist schematisch ein Messaufbau 100 gezeigt, der erfindungsgemäß zur Ermittlung der Ultraschallaufzeit eines Ultraschallsignals in einer Spannbetonschwelle 1 genutzt werden kann. Das Bezugszeichen 2 bezeichnet das Ankopplungsmedium, beispielsweise eine als Prüfflüssigkeit verwendete wässrige Lösung, z.B. Wasser. Das Bezugszeichen 3.1 steht für den Ultraschallprüfkopf (Sender); das Bezugszeichen 3.2 bezeichnet den zweiten Ultraschallprüfkopf (Ultraschallsensor bzw. Empfänger) der dargestellten zwei Paare; das Bezugszeichen 8 steht für die Spannstähle der Spannbetonschwelle; $l_t$ steht für die Gesamtlänge der Durchschallungsstrecke; $l_s$ steht für die Länge der Durchschallungsstrecke in der Spannbetonschwelle - hier am Beispiel des obersten Paares Ultraschallprüfköpfe gezeigt; $l_{c1} + l_{c2}$ ist die Länge der Durchschallungsstrecke des Ankopplungsmediums ($l_c$ - die summarische Länge der Durchschallungsstrecke im Ankopplungsmedium) für die oberste Durchschallungsstrecke. Der gestrichelte, jeweils vom Sender 3.1 zum Empfänger 3.2 gerichtete Pfeil symbolisiert ein entlang der Durchschallungsachse gesendetes Ultraschallsignal. Als Ultraschallsignale kommen Pulse im 50 - 500 kHz, bevorzugt im Bereich 100 kHz - 300 kHz, weiter bevorzugt 150 kHz - 200 kHz.

Typische Bereiche liegen zwischen 10 - 200 Microsekunden ($\mu$s) Dauer, bevorzugt im Bereich 20 - 100 $\mu$s, weiter bevorzugt im Bereich 20 -40 $\mu$s. Eine bevorzugte Pulsform sind Sinuswellen, die Frequenz wird entsprechend der Eigenfrequenz der Prüfköpfe (Resonanz) gewählt, Pulsdauer 1 - 6 volle Schwingungen; die Amplitude der Anregungsspannung entspricht der technischen Spezifikationen der Prüfköpfe. Bei den hier verwendeten Prüfköpfen der Fa. Vallen waren das beispielsweise 450 V$_{PP}$.

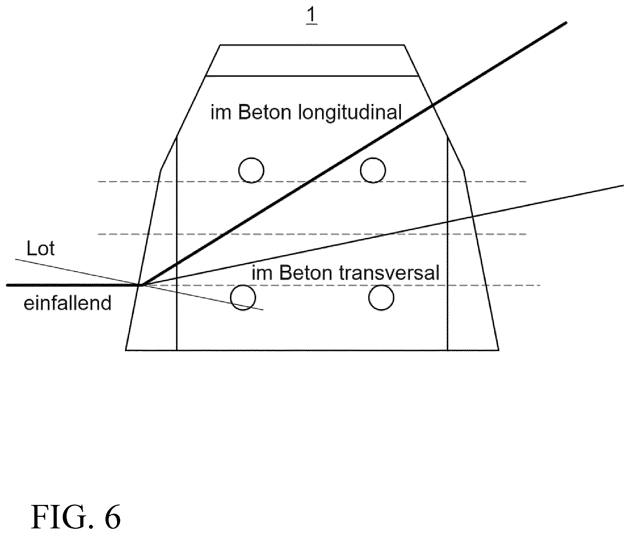

**Fig. 6** zeigt schematisch die vom Einschallwinkel abhängige Ausbreitungsrichtung von Longitudinal- und von korrespondierenden Transversalwellen im beschallten Beton der Schwelle 1, wobei die Ausbreitungsgeschwindigkeit der Longitudinalwelle naturgemäß über jener der Transversalwelle liegt.

Wie in **Fig. 14** detaillierter dargestellt ist, wird der Einschallwinkel $\vartheta_a$ zu einer Oberflächennormalen der Seitenfläche 1$_S$ der Spannbetonschwelle 1 in den jeweiligen Messpositionen typischerweise gemäß folgender Gleichung bestimmt:

$$\sin \vartheta_a / c_a = \sin \vartheta_b / c_b$$

mit der Schallgeschwindigkeit $c_b$ des (Ultraschall-) Durchschallungssignals im Beton der Spannbetonschwelle 1, der

Schallgeschwindigkeit $c_a$ des Ultraschallsignals im Ankopplungsfluids und dem Winkel $\vartheta_b$ der Oberflächennormalen der exemplarischen linken Seitenfläche $1_S$ der Spannbetonschwelle 1, durch die das Durchschallungssignal in die Schwelle 1 eintritt, bezüglich einer Grundfläche $1_G$ der Spannbetonschwelle 1, einer Deckfläche $1_D$ der Spannbetonschwelle 1 bzw. einer Ebene E, in der typischerweise sowohl die Transversalwelle als auch die Longitudinalwelle des Durchschallungssignals die Spannbetonschwelle 1 in der gezeigten Messposition durchlaufen (roter Pfeil).

In dem exemplarischen Ausführungsbeispiel mit $\vartheta_b$ = 11° sind (aus Symmetriegründen) der Einfallswinkel $\vartheta_a$ und der Ausfallwinkel des Ultraschallsignals an der gegenüberliegenden Seitenfläche $1_{S'}$ der Spannbetonschwelle 1 gleich groß und betragen jeweils etwa 3°.

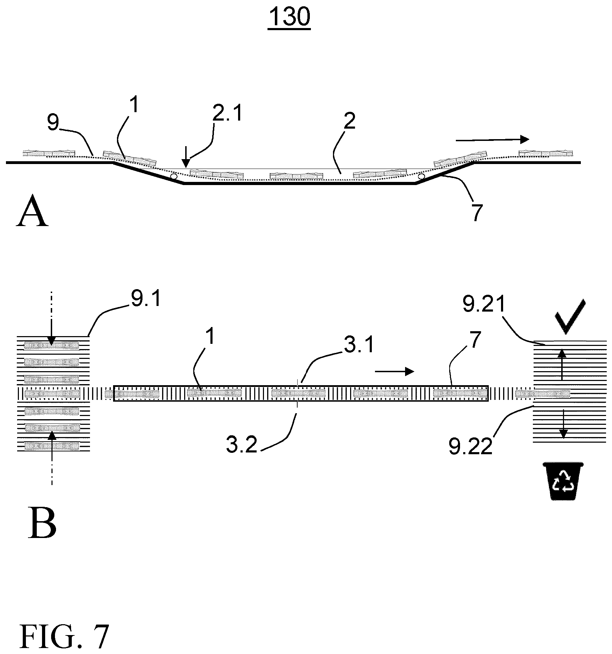

**Fig. 7** zeigt ein System 110 zur kontinuierlichen (Durchlauf-)Prüfung einer Vielzahl von Spannbetonschwellen 1. In **Fig. 7A** ist eine Seitenansicht der Anlage 110 gezeigt, umfassend ein Transportband 9 auf dem die Spannbetonschwellen 1 kontinuierlich durch den Prüfbehälter 7 der das in diesem Fall flüssige Ankopplungsmedium 2, bevorzugt Wasser 2, enthält. **Fig. 7B** zeigt eine Draufsicht der Anlage 110 mit einem orthogonal zur Laufrichtung des ersten Transportbandes 9 ausgerichteten Rollenfeld oder zweiten Transportband 9.1, mit dem die zu prüfenden Schwellen 1 ausgerichtet und auf das erste Transportband 9 geschoben werden. Zusätzlich verwendbare Leitplanken und Aktoren sind nicht dargestellt. Pfeile zeigen die Laufrichtungen der Transportbänder an. Die am Ende des ersten Transportbandes 9 angeordnete Gruppe eines dritten Transportbandes 9.21 und eines vierten Transportbandes 9.22 dient zum Abtrennen der durch das Prüfkopfpaar umfassend die Ultraschallprüfköpfe 3.1 und 3.2 als untauglich befundenen Spannbetonschwellen 1 von den als tauglich bewerteten. Ein entsprechend gesteuerter Aktor (nicht dargestellt) bestimmt die weitere Bewegungsrichtung der geprüften Spannbetonschwellen in Abhängigkeit vom jeweils erhaltenen Prüfbefund. Aus Gründen der Übersichtlichkeit ist nur ein Paar Prüfköpfe 3.1 und 3.2 dargestellt, auch sind sämtliche weiteren fachüblich verwendeten Anlagenbestandteile, z.B. Kontroll- und Steuereinheit, Speicher, Impulsgeber, Spannungsquelle usw. nicht dargestellt.

Vorteilhaft ermöglicht die vorgeschlagene Anordnung eine zuverlässige Durchschallung individueller Spannbetonschwellen und hat damit folgende Vorteile:

- objektive Prüfung;
- Zeitersparnis;
- nachhaltige, rückführbare Dokumentation der Prüfergebnisse;
- integrale automatisierte Prüfung;
- Feststellen der Art der vorliegender Inhomogenitäten;
- Kostenvorteil,
- Präzision;
- Messabstand in Längsrichtung: 1-10 mm;
- die Auflösung des Nachweises von Gefügestörungen liegt im Bereich der Wellenlänge (ca. 3 cm bei 5000m/s und 150 kHz);
- Durchschallung erfolgt bevorzugt in 4 Ebenen.

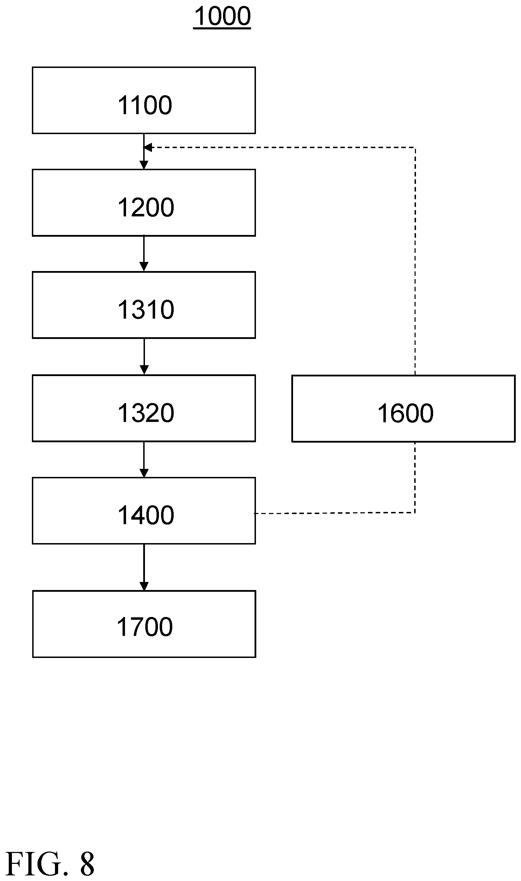

**Fig. 8** zeigt schematisch die Abfolge der einzelnen, hier schematisch als Blöcke dargestellten Schritte 1100, 1200, 1310, 1320, 1400, und 1700 des vorgeschlagenen Verfahrens 1000 zur Bewertung und/oder - im Fall des Einhaltens des zuvor an Ultraschalllaufzeiten einer neuwertigen Spannbetonschwelle definierten Schwellenwertes - einer Zertifizierung einer (gebrauchten) Spannbetonschwelle für deren (erneuten) Einsatz als Gleisschwelle in einem Gleisbett. Ist die Spannbetonschwelle in der Messanordnung 100, 110 zwischen den Ultraschallprüfköpfen 3.1 und 3.2 angeordnet und ist vollständig, mindestens mit 6 cm Wasser überdeckt. Dabei beträgt ein Abstand der Prüfköpfe zur Schwelle: 0,5 cm bis 10 cm, liegt bevorzugt in einem Bereich von 0,5 cm bis 1 cm, sodass die Schwelle vom Ankopplungsfluid 2 vollständig umgeben ist. Erst dann schließt sich die erfindungsgemäß bezeichnete Abfolge der einzelnen Schritte des vorgeschlagenen Verfahrens 1000 an. Diese Schritte sind somit zumindest: "Positionieren 1200 eines Paares oder mehrerer Paare von Ultraschallprüfköpfen 3, 3.1, 3.2,..."; "Aussenden 1310 eines Ultraschallsignals durch einen als Sender 3.1 konfigurierten Ultraschallprüfkopf des Paares Ultraschallprüfköpfe 3"; "Ermitteln 1400 einer Ultraschalllaufzeit ($t_s$) des ausgesendeten Ultraschallsignals innerhalb der Spannbetonschwelle 1". Diese Sequenz wird für jede weitere Messposition wiederholt: Jeweils danach oder nach dem Durchlaufen eines vollständigen Messzyklus an allen Messpositionen entlang der Spannbetonschwelle 1 folgt der Schritt "Vergleichen 1500 der ermittelten Ultraschalllaufzeit/en ($t_s$) mit einem Schwellenwert der Ultraschalllaufzeit/en" Zweckmäßigerweise kann eine gleitende Mittelwertbildung über so viele benachbarte Messpositionen erfolgen, dass das höchstens einer halben Wellenlänge entspricht, also bei einem bevorzugten Messpunktabstand von 1 mm und 150 kHz eine Mittelwertbildung über 15 Messwerte, bevorzugt über 5 Messwerte erfolgt und sodann ein abschließendes "Bewerten 1700 der Spannbetonschwelle 1 als *"tauglich"* oder als *"untauglich"* für den Einsatz als Gleisschwelle in einem Gleisbett" das Verfahren abschließt. Wie durch den gestrichelten Pfeil in **Fig. 8** dargestellt wird, weist das Verfahren 1000 typischerweise ein Verschieben 1600 des Paares Ultraschallprüfköpfe 3, 3.1, 3.2... aus der aktuellen Messposition relativ zu der Lage eines markanten Bezugspunktes 1.x oder einer Bezugsgeraden der zu prüfenden Spannbetonschwelle 1 in eine weitere Messposition auf, insbesondere bis alles Messpositionen durchlaufen sind und/oder wenn die ermittelte Ultraschalllaufzeit in (wenigstens einer, typischer mehreren) der aktuellen Messposition oberhalb des Schwellenwertes dafür liegt.

Typischerweise umfasst die zum Positionieren der zu prüfenden Spannbetonschwelle 1 eingerichtete Messanordnung 100, 110 ein Transportband oder eine Rollenstrecke 9 (vgl. **Fig. 7**) , die eine kontinuierliche und bevorzugt - zumindest im Bereich zwischen den Sende- und Empfänger-Messköpfen - mit gleichbleibender Geschwindigkeit erfolgende Bewegung und kontinuierliche Nachführung weiterer zu prüfender Spannbetonschwellen 1 gewährleistet.

Dies kann über die Nutzung einer Transportkette 9 oder eines Transportbandes 9 mit entsprechenden Mitnehmern, insbesondere mit Anschlags- und Vortriebshaken, vergleichbar zu einer Transportkette in einer Autowaschstraße, erfolgen. Abweichend vom Transport der Autos in der Autowaschstraße wird das entsprechende Transportband hier jedoch durch ein mit dem Ankopplungsfluid 2 gefülltes Becken 7 geführt, bevorzugt am Boden des Beckens 7, das mit dem Kopplungsmedium 2 gefüllt ist, wenn das Kopplungsfluid 2 eine wässrige Lösung, insbesondere Wasser 2 ist. Sinnvollerweise sind mehrere Paare von Ultraschallprüfköpfen (Sender und Empfänger, 3.1, 3.2) in einem festen Abstand entlang des Beckens so angeordnet, dass beim einmaligen Vorbeiführen der Spannbetonschwelle 1 deren vollständige Durchschallung und damit vollständige Prüfung gewährleistet ist. Wie in Fig. 2 gezeigt, kann die Durchschallung nicht nur horizontal, sondern auch in vertikaler Richtung - bezogen auf die Betriebsposition der Spannbetonschwelle im Gleisbett - erfolgen.

Dazu können mehrere Paare Ultraschallprüfköpfe 3.1, 3.2 ....in Form eines Arrays vertikal versetzt angeordnet sein. Zwei grundlegend unterschiedliche Messmoden sind anwendbar: Entweder ein vollständiges Prüfen durch Positionieren der Schwelle an einem oder nur wenigen "Haltepunkten" in Kombination mit besagten arrayartig angeordneten Prüfkopfpaaren, oder kontinuierliches Vorbeiführen der zu prüfenden Schwelle an zeilenartig übereinander, ggf. ebenfalls in Form eines Arrays angeordneten Prüfkopfpaaren.

Einzelne Aspekte der vorstehenden Beschreibung lassen sich beispielhaft zusammenfassen als:

1. Ultraschallprüfung an Spannbetonschwellen im Wasserbad.

2. Automatisches Anfahren einer reproduzierbar einstellbaren Position eines Ultraschallprüfkopfes (Sender und/oder Empfänger) relativ zu einem Bezugspunkt oder einer Bezugsgeraden der Spannbetonschwelle.

3. Automatisches Verknüpfen einer gewählten Messposition mit dem entsprechenden Messwert der an dieser Messposition der Ultraschall-Prüfköpfe relativ zur Spannbetonschwelle ermittelten Ultraschalllaufzeit, d.h. der Laufzeit des Ultraschallsignals im Gefüge der Spannbetonschwelle.

4. Automatisches Erkennen einer Gefüge-Störung an Hand der aktuell mit einem kalibrierten Messaufbaus gemessenen Ultraschalllaufzeit.

5. Automatisches Erkennen einer Größe (räumlichen Ausdehnung) und Position einer Gefüge-Störung in einer Spannbetonschwelle.

6. Automatisches Bewerten einer Spannbetonschwelle anhand von Vorgaben für Toleranzen oder Schwellenwerten für Gefüge-Störungen, wobei jeweilige Toleranzbereiche eine Größe (räumliche Ausdehnung) und eine Position der tolerierbaren Gefüge-Störungen definieren und Schwellenwerte eine maximale Laufzeitdauer eines Ultraschallsignals nach oben begrenzen, also nicht überschritten werden dürfen.

Übersteigt ein Wert der gemessenen Ultraschalllaufzeit einen als Schwellenwert an der entsprechenden Position definierten Wert, gilt das Gefüge an dieser Messposition als gestört und disqualifiziert die Spannbetonschwelle als ungeeignet für eine Wiederverwendung in einem Gleisbett.

Zur Interpretation erhaltener Messwerte können Bezugspunkte auf einer Oberfläche oder im Inneren der zu vermessenden Spannbetonschwelle herangezogen werden. Als derartige Referenzpunkte kommen visuell und/oder idealerweise messtechnisch eineindeutig identifizierbare Strukturen in Betracht, beispielsweise eine Sicke 12, eine Sickenschulter 13, eine Flankenfläche der Spannbetonschwelle oder einer, der in der Spannbetonschwelle angeordneten Dübel D1.A, D1.I, D2.I, D2.A. Als mögliche Bezugsgeraden sind beispielsweise ein Winkel, eine Kante oder eine gerade Linie entlang einer Flanke der Spannbetonschwelle geeignet. Die Verwendung von Bezugspunkten und/oder -geraden ermöglicht eine reproduzierbar und damit standardisierbar durchführbare Prüfung von Spannbetonschwellen 1 auf ihre Wiedereinsetzbarkeit in einem Gleisbett.

Unter dem Begriff Spannbetonschwelle werden vorliegend hauptsächlich Gleisschwellen der nachfolgend bezeichneten Typen subsummiert: B 70 W-(60 und 54), B 70 Wo-(60 und 54); B 70 So W-(60 und 54), B 70 W-2,4 (60 und 54); B 75 W-300; B 90 W-(60 und 54); B 90 So W-(60 und 54); BBS 1 W-(60 und 54); B 01 W-60; B 07 W-60; B 70 W54-BS-D HH; B 70 W54-BS-D B; B 70 W54-bs-G; FS 150; ZSX; B6; B 9; B 91; B 61, B 62; B12; B 121; B 53; BV 53; B 55 K; BS 55; BS 60; B 58 K; BS 62; BS 65; BS 66; BS 78; B 58 W-54; BoS 4 i; BoS 6 i; B 90 W-54 (B und HH) sowie BoSW-54 (B und HH); wobei Einzelheiten zu den benannten Typen beispielsweise veröffentlicht sind in: "Betonschwellen Feste Fahrbahn Fertigteiltragplatten Komponenten im Netz der Deutschen Bahn AG" Hrsg.: Betonschwellenindustrie e.V.; 2. Aufl. 2017.

Zusammenfassend wird erfindungsgemäß somit ein zerstörungsfreies Prüfverfahren zur Beurteilung der Einsetzbarkeit einer Spannbetonschwelle (1) als Gleisschwelle in einem Gleisbett für ein Eisenbahnschienennetz vorgeschlagen, aufweisend:

a. Anordnen (1100) der zu prüfenden Spannbetonschwelle (1) in einem Ankopplungsfluid (2);

b. Positionieren (1200) eines Paares von Ultraschallprüfköpfen (3) beidseitig der zu prüfenden Spannbetonschwelle (1) in einem jeweiligen definierten Abstand (lc1, lc2 ) und in einem jeweilig definierten Winkel zu Oberflächen der Spannbetonschwelle (1) in mindestens einer Messposition;

c. Aussenden (1310) eines Ultraschallsignals durch einen als Sender (3.1) konfigurierten Ultraschallprüfkopf des Paares von Ultraschallprüfköpfen (3) und Empfangen (1320) eines Durchschallungssignals durch einen als Empfänger (3.2) konfigurierten Ultraschallprüfkopf des Paares von Ultraschallprüfköpfen (3), wobei das Aussenden (1310) und das Empfangen (1320) in der mindestens einen Messposition erfolgt;

d. Ermitteln (1400) einer Ultraschalllaufzeit ($t_s$) des in der mindestens einen Messposition ausgesendeten und empfangenen Ultraschallsignals im Beton der Spannbetonschwelle (1);

e. Bewerten (1700) der Spannbetonschwelle (1) als tauglich für die Verwendung als Gleisschwelle im Gleisbett, wenn die ermittelte Ultraschalllaufzeit in jeder Messposition der mindestens einen Messposition unterhalb eines jeweiligen Schwellenwertes liegt oder dem jeweiligen Schwellenwert entspricht, und als untauglich für die Verwendung der Spannbetonschwelle (1) als Gleisschwelle im Gleisbett, wenn die ermittelte Ultraschalllaufzeit in wenigstens einer Messposition der mindestens einen Messposition oberhalb des jeweiligen Schwellenwertes liegt.

Weiter wird ein System (100, 110, 120, 130) zur Prüfung und/oder Zertifizierung einer Spannbetonschwelle (1) mit dem bezeichneten zerstörungsfreien Prüfverfahren (10000) vorgeschlagen, sowie dessen verfahrensgerechte Verwendung sowie die Wiederverwendung einer im zerstörungsfreien Prüfverfahren (1000) als tauglich befundenen Spannbetonschwelle (1) in einem Gleisbett.

Die vorliegende Erfindung wurde anhand von Aspekten und Ausführungsbeispielen erläutert. Diese Ausführungsbeispiele sollten keinesfalls als einschränkend für die vorliegende Erfindung verstanden werden. Die nachfolgenden Patentansprüche stellen einen ersten, nicht bindenden Versuch dar, die Erfindung allgemein zu definieren.

AUSFÜHRUNGSFORMEN

1. Verfahren (1000) zur Prüfung von Spannbetonschwellen (1) auf eine Verwendbarkeit als Gleisschwelle in einem Gleisbett, aufweisend:

a. Anordnen (1100) der zu prüfenden Spannbetonschwelle (1) in einem Ankopplungsfluid (2);

b. Positionieren (1200) eines Paares von Ultraschallprüfköpfen (3) beidseitig der zu prüfenden Spannbetonschwelle (1) in einem jeweiligen definierten Abstand ($l_{c1}$ , $l_{c2}$ ) und in einem jeweilig definierten Winkel zu Oberflächen der Spannbetonschwelle (1) in mindestens einer Messposition;

c. Aussenden (1310) eines Ultraschallsignals durch einen als Sender (3.1) konfigurierten Ultraschallprüfkopf des Paares von Ultraschallprüfköpfen (3) und Empfangen (1320) eines Durchschallungssignals durch einen als Empfänger (3.2) konfigurierten Ultraschallprüfkopf des Paares von Ultraschallprüfköpfen (3), wobei das Aussenden (1310) und das Empfangen (1320) in der mindestens einen Messposition erfolgt;

d. Ermitteln (1400) einer Ultraschalllaufzeit ($t_s$) des in der mindestens einen Messposition ausgesendeten und empfangenen Ultraschallsignals im Beton der Spannbetonschwelle (1);

e. Bewerten (1700) der Spannbetonschwelle (1) als tauglich für die Verwendung als Gleisschwelle im Gleisbett, wenn die ermittelte Ultraschalllaufzeit in jeder Messposition der mindestens einen Messposition unterhalb eines jeweiligen Schwellenwertes liegt oder dem jeweiligen Schwellenwert entspricht, und als untauglich für die Verwendung der Spannbetonschwelle (1) als Gleisschwelle im Gleisbett, wenn die ermittelte Ultraschalllaufzeit in wenigstens einer Messposition der mindestens einen Messposition oberhalb des jeweiligen Schwellenwertes liegt.

2. Verfahren (1000) nach Ausführungsform 1,
wobei das Ankopplungsfluid (2) im Schritt Anordnen (1100) ausgewählt ist unter einer Flüssigkeit, insbesondere ausgewählt ist unter einer wässrigen Lösung und Wasser.

3. Verfahren nach einer der vorstehenden Ausführungsformen,
wobei das Ermitteln der Ultraschalllaufzeit im Schritt d. aus einer Differenz eines Zeitpunktes des Aussendens des Ultraschallsignals und eines Zeitpunktes des Empfangens des Durchschallungssignals unter Berücksichtigung einer Länge einer Ankopplungsfluid-Durchschallungsstrecke ($l_{c1}$, $l_{c2}$) im verwendeten Ankopplungsfluid (2) und einer Länge einer Beton-Durchschallungsstrecke ($l_s$) im Beton der Spannbetonschwelle (1) erfolgt.

4. Verfahren nach einer der vorstehenden Ausführungsformen, weiter umfassend:

f. Anordnen (1200) des Paares Ultraschallprüfköpfe (3, 3.1, 3.2...) in der mindestens einen Messposition mit Bezug zu einem markanten Bezugspunkt der Spannbetonschwelle (1),
und/oder

g. Verschieben (1600) des Paares Ultraschallprüfköpfe (3, 3.1, 3.2...) aus einer Messposition der mindestens einen Messposition relativ zu der Lage des markanten Bezugspunktes (1.x) oder einer Bezugsgeraden der zu prüfenden Spannbetonschwelle (1) in eine weitere Messposition der mindestens einen Messposition.

5. Verfahren (1000) nach Ausführungsform 4,

wobei das Anordnen gemäß Schritt f. und/oder Verschieben gemäß Schritt g. ein automatisches Bewegen der Spannbetonschwelle (1) aus einer vorausgehenden Position umfasst,

wobei das automatische Bewegen der Spannbetonschwelle insbesondere kontinuierlich oder diskontinuierlich erfolgt.

6. Verfahren nach Ausführungsform 5,
wobei das automatische Bewegen der Spannbetonschwelle (1) diskontinuierlich erfolgt und das Aussenden (1310) und das Empfangen (1320) von Ultraschallsignalen gemäß Schritt c. während eines Verweilens der Spannbetonschwelle (1) in einer jeweils eingenommenen Messposition erfolgt.

7. Verfahren (1000) nach Ausführungsform 6,
wobei das automatische Bewegen der Spannbetonschwelle (1) kontinuierlich erfolgt und das Aussenden (1310) und das Empfangen (1320) von Ultraschallsignalen gemäß Schritt c. in einem so kurzen Zeitraum erfolgt, dass die Messung trotz der Bewegung der Spannbetonschwelle (1) einer jeweils eingenommenen Messposition zuordenbar ist.

8. Verfahren (1000) nach Ausführungsform 7,
wobei das automatische Bewegen mit einer Geschwindigkeit von 0.5 cm/s bis 2,5 cm/s, insbesondere mit einer Geschwindigkeit von 1 cm/s erfolgt und die Pulsfolgefrequenz $\leq$ 10 Hz ist.

9. Verfahren (1000) nach einer der vorstehenden Ausführungsformen,
wobei die verwendeten Ultraschall-Prüfköpfe (3, 3.1, 3.2) für einen Frequenzbereich von 30 kHz bis 1000 kHz, bevorzugt für einen Bereich von 100 kHz bis 500 kHz, weiter bevorzugt für einen Bereich zwischen 100 kHz bis 200 KHz ausgelegt sind.

10. Verfahren (1000) nach einer der vorstehenden Ausführungsformen,
wobei eine als Pulsfolgefrequenz definierte Abfolge von aufeinanderfolgend ausgesendeten Ultraschallpulsen einen Wert von 20 Hz nicht übersteigt, insbesondere die Pulsfolgefrequenz $\leq$ 10 Hz ist.

11. Verfahren (1000) nach einer der vorstehenden Ausführungsformen, weiter umfassend:

h. Definieren der mindestens einen Messposition als innerhalb eines Bereichs der Einleitung einer Vorspannkraft in den Beton der Spannbetonschwelle (1) liegend.

12. Verfahren (1000) nach einer der vorstehenden Ausführungsformen,

wobei die Spannbetonschwelle (1) eine zuvor gebrauchte und einem Gleisbett entnommene Spannbetonschwelle (1) ist.

13. Verfahren (1000) nach einer der vorstehenden Ausführungsformen,

wobei der jeweils definierte Abstand ($l_{c1}$, $l_{c2}$) gemäß Schritt b. in einem Bereich von 0.5 cm bis 10 cm, bevorzugt in einem Bereich von 0.5 cm bis 1 cm liegt.

14. Verfahren (1000) nach einer der vorstehenden Ausführungsformen,

wobei das Positionieren des Paares von Ultraschallprüfköpfen (3) gemäß Schritt b. ein einer äußeren Kontur der Spannbetonschwelle (1) folgendes Nachführen zumindest des als Empfänger (3.2) konfigurierten Ultraschallprüfkopf (3) des Paares von Ultraschallprüfköpfen (3) umfasst.

15. Verfahren (1000) nach einer der vorstehenden Ausführungsformen,

wobei der jeweils definierte Winkel gemäß Schritt b. ausgedrückt als Winkel ($\vartheta_a$) zu einer Oberflächennormalen der Oberfläche ($1_S$, $1_{S'}$) der Spannbetonschwelle (1) in den Messpositionen gemäß der Gleichung:

$$\sin \vartheta_a / c_a = \sin \vartheta_b / c_b$$

bestimmt wird,

wobei $c_b$ die Schallgeschwindigkeit im Beton der Spannbetonschwelle (1) ist, $c_a$ die Schallgeschwindigkeit im Ankopplungsfluid ist, und $\vartheta_b$ ein Winkel der Oberflächennormalen der Oberfläche ($1_S$) der Spannbetonschwelle (1) bezüglich einer Grundfläche ($1_G$) der Spannbetonschwelle (1), einer Deckfläche ($1_D$) der Spannbetonschwelle (1) und/oder einer Ebene (E) ist, in der das durch den als Sender (3.1) konfigurierten Ultraschallprüfkopf des Paares von Ultraschallprüfköpfen (3) ausgesandte Ultraschallsignal die Spannbetonschwelle (1) in der jeweiligen Messposition die Spannbetonschwelle (1) durchlaufen soll.

16. Verfahren (1000) nach einem der vorstehenden Ausführungsformen,

wobei der jeweils definierte Winkel gemäß Schritt b. so gewählt ist, dass eine Ausbreitungsrichtung einer durch das Aussenden des Ultraschallsignals im Beton der Spannbetonschwelle generierten Transversalwelle mit einer im Beton der Spannbetonschwelle (1) generierten Longitudinalwelle weitestgehend zusammenfallen.

17. Verfahren (1000) nach einer der vorstehenden Ausführungsformen, wobei das Anordnen (1100) gemäß Schritt a. umfasst:

a1. Entnehmen der Spannbetonschwelle (1) aus dem Gleisbett;

a2. Reinigen der entnommenen Spannbetonschwelle (1) von oberflächlichen Verunreinigungen, und/oder

a3. Überführen der gereinigten Spannbetonschwelle (1) in einen Prüfbehälter (7) umfassend das Ankopplungsfluid (2).

18. Verfahren (1000) nach einer der vorstehenden Ausführungsformen, weiter umfassend:

Bestimmen des jeweiligen Schwellenwertes für die Ultraschalllaufzeit in der mindestens einen Messposition mittels einer Vergleichsmessung an einer Vergleichsspannbetonschwelle, typischerweise mittels Vergleichsmessungen an mehreren Vergleichsspannbetonschwellen und/oder mittels einer numerischen Simulation der Schallausbreitung in einem Modell für die Spannbetonschwelle.

19. System (100, 110, 120, 130) zur Prüfung einer Spannbetonschwelle (1) für deren Verwendbarkeit in einem Gleisbett, umfassend:

- zumindest ein Paar Ultraschallprüfköpfe (3, 3.1, 3.2...);
- ein Ankopplungsmedium;
- ein Bewegungsmittel (9), ausgewählt unter einem Prüfkopfbewegungsmittel (9) und einem Spannbetonschwellenbewegungsmittel (9); und
- eine Kontroll- und Steuereinheit, die eingerichtet ist, das zumindest eine Paar Ultraschallprüfköpfe (3, 3.1, 3.2...) und das Bewegungsmittel (9) so anzusteuern, dass das System ein Verfahren (1000) nach einem der vorhergehenden Ansprüche ausführt.

20. System (100, 110, 120, 130) nach Ausführungsform 19,
wobei ein Abstand zwischen in Längsrichtung der Spannbetonschwelle (1) zur Ermittlung der Folge von Ultraschalllaufzeiten eingenommenen jeweiligen Messpositionen in einem Bereich von 1 - 100 mm, bevorzugt in dem Bereich von 2 bis 10 mm liegt.

21. System (100, 110, 120, 130) nach einer der Ausführungsformen 19 bis 20,

wobei das Bewegungsmittel (9) eingerichtet ist zur reproduzierbaren Positionierung des Paares Ultraschall-Prüfköpfe (3, 3.1, 3.2) an einer Vielzahl von Messpositionen, die in einer Ebene liegen, die orthogonal zu einer Durchschallungsachse ausgerichtet ist, und

wobei die Durchschallungsachse parallel einer Längsachse der Spannbetonschwelle (1) oder orthogonal zu einer Ebene, die durch die Längsachse der Spannbetonschwelle (1) definiert wird, orientiert ist.

22. Verwendung einer Messanordnung zur Bestimmung einer Ultraschalllaufzeit in einem Festkörper für eine Prüfung einer Spannbetonschwelle (1) auf deren Verwendbarkeit in einem Gleisbett,
wobei die Messanordnung ein System (100, 110, 120, 130) nach einem der Ansprüche 19 bis 21 umfasst.

23. Verwendung nach Ausführungsform 22,
wobei mindestens ein weiteres Paar Ultraschallprüfköpfe (3, 3.1, 3.2) so angeordnet ist, dass die Signale der verschiedenen Prüfkopfpaare (3) sich nicht gegenseitig beeinflussen und so eine gleichzeitige Prüfung der Spannbetonschwelle (1) in verschiedenen Messebenen (Messpositionen) möglich ist.

24. Verwendung einer nach einem Verfahren gemäß einer der Ausführungsformen 1- 18 als tauglich bewerteten Spannbetonschwelle (1) als Gleisschwelle in einem Gleisbett.

**Patentansprüche**

1. Verfahren (1000) zur Prüfung von Spannbetonschwellen (1) auf eine Verwendbarkeit als Gleisschwelle in einem Gleisbett, aufweisend:

a. Anordnen (1100) der zu prüfenden Spannbetonschwelle (1) in einem Ankopplungsfluid (2);
b. Positionieren (1200) eines Paares von Ultraschallprüfköpfen (3) beidseitig der zu prüfenden Spannbetonschwelle (1) in einem jeweiligen definierten Abstand ($l_{c1}$, $l_{c2}$) und in einem jeweilig definierten Winkel zu Oberflächen der Spannbetonschwelle (1) in mindestens einer Messposition;
c. Aussenden (1310) eines Ultraschallsignals durch einen als Sender (3.1) konfigurierten Ultraschallprüfkopf des Paares von Ultraschallprüfköpfen (3) und Empfangen (1320) eines Durchschallungssignals durch einen als Empfänger (3.2) konfigurierten Ultraschallprüfkopf des Paares von Ultraschallprüfköpfen (3), wobei das Aussenden (1310) und das Empfangen (1320) in der mindestens einen Messposition erfolgt;
d. Ermitteln (1400) einer Ultraschalllaufzeit ($t_s$) des in der mindestens einen Messposition ausgesendeten und empfangenen Ultraschallsignals im Beton der Spannbetonschwelle (1);
e. Bewerten (1700) der Spannbetonschwelle (1) als tauglich für die Verwendung als Gleisschwelle im Gleisbett, wenn die ermittelte Ultraschalllaufzeit in jeder Messposition der mindestens einen Messposition unterhalb eines jeweiligen Schwellenwertes liegt oder dem jeweiligen Schwellenwert entspricht, und als untauglich für die Verwendung der Spannbetonschwelle (1) als Gleisschwelle im Gleisbett, wenn die ermittelte Ultraschalllaufzeit in wenigstens einer Messposition der mindestens einen Messposition oberhalb des jeweiligen Schwellenwertes liegt;

wobei das Ankopplungsfluid (2) im Schritt Anordnen (1100) ausgewählt ist unter einer Flüssigkeit, insbesondere ausgewählt ist unter einer wässrigen Lösung und Wasser.

2. Verfahren nach Anspruch 1,
wobei das Ermitteln der Ultraschalllaufzeit im Schritt d. aus einer Differenz eines Zeitpunktes des Aussendens des Ultraschallsignals und eines Zeitpunktes des Empfangens des Durchschallungssignals unter Berücksichtigung einer Länge einer Ankopplungsfluid-Durchschallungsstrecke ($l_{c1}$, $l_{c2}$) im verwendeten Ankopplungsfluid (2) und einer Länge einer Beton-Durchschallungsstrecke ($l_{s}$) im Beton der Spannbetonschwelle (1) erfolgt.

3. Verfahren nach einem der vorstehenden Ansprüche, weiter umfassend:

   f. Anordnen (1200) des Paares Ultraschallprüfköpfe (3, 3.1, 3.2...) in der mindestens einen Messposition mit Bezug zu einem markanten Bezugspunkt der Spannbetonschwelle (1),
   und/oder
   g. Verschieben (1600) des Paares Ultraschallprüfköpfe (3, 3.1, 3.2...) aus einer Messposition der mindestens einen Messposition relativ zu der Lage des markanten Bezugspunktes (1.x) oder einer Bezugsgeraden der zu prüfenden Spannbetonschwelle (1) in eine weitere Messposition der mindestens einen Messposition;

   wobei das Anordnen gemäß Schritt f. und/oder das Verschieben gemäß Schritt g. ein automatisches kontinuierliches oder diskontinuierliches Bewegen der Spannbetonschwelle (1) aus einer vorausgehenden Position umfasst.

4. Verfahren nach Anspruch 3,
wobei das automatische Bewegen der Spannbetonschwelle (1) diskontinuierlich erfolgt und das Aussenden (1310) und das Empfangen (1320) von Ultraschallsignalen gemäß Schritt c. während eines Verweilens der Spannbetonschwelle (1) in einer jeweils eingenommenen Messposition erfolgt.

5. Verfahren (1000) nach Anspruch 3,

   wobei das automatische Bewegen der Spannbetonschwelle (1) kontinuierlich erfolgt und das Aussenden (1310) und das Empfangen (1320) von Ultraschallsignalen gemäß Schritt c. in einem so kurzen Zeitraum erfolgt, dass die Messung trotz der Bewegung der Spannbetonschwelle (1) einer jeweils eingenommenen Messposition zuordenbar ist; und
   wobei das automatische Bewegen mit einer Geschwindigkeit von 0.5 cm/s bis 2,5 cm/s, insbesondere mit einer Geschwindigkeit von 1 cm/s erfolgt und die Pulsfolgefrequenz $\leq$ 10 Hz ist.

6. Verfahren (1000) nach einem der vorstehenden Ansprüche,

   wobei die verwendeten Ultraschall-Prüfköpfe (3, 3.1, 3.2) für einen Frequenzbereich von 30 kHz bis 1000 kHz, bevorzugt für einen Bereich von 100 kHz bis 500 kHz, weiter bevorzugt für einen Bereich zwischen 100 kHz bis 200 KHz ausgelegt sind; und
   wobei optional eine als Pulsfolgefrequenz definierte Abfolge von aufeinanderfolgend ausgesendeten Ultraschallpulsen einen Wert von 20 Hz nicht übersteigt, insbesondere die Pulsfolgefrequenz $\leq$ 10 Hz ist.

7. Verfahren (1000) nach einem der vorstehenden Ansprüche, weiter umfassend:
h. Definieren der mindestens einen Messposition als innerhalb eines Bereichs der Einleitung einer Vorspannkraft in den Beton der Spannbetonschwelle (1) liegend.

8. Verfahren (1000) nach einem der vorstehenden Ansprüche,
wobei der jeweils definierte Abstand ($l_{c1}$, $l_{c2}$) gemäß Schritt b. in einem Bereich von 0.5 cm bis 10 cm, bevorzugt in einem Bereich von 0.5 cm bis 1 cm liegt.

9. Verfahren (1000) nach einem der vorstehenden Ansprüche,
wobei das Positionieren des Paares von Ultraschallprüfköpfen (3) gemäß Schritt b. ein einer äußeren Kontur der Spannbetonschwelle (1) folgendes Nachführen zumindest des als Empfänger (3.2) konfigurierten Ultraschallprüfkopf (3) des Paares von Ultraschallprüfköpfen (3) umfasst.

10. Verfahren (1000) nach einem der vorstehenden Ansprüche,

   wobei der jeweils definierte Winkel gemäß Schritt b. ausgedrückt als Winkel ($\vartheta_a$) zu einer Oberflächennormalen

der Oberfläche ($1_S$, $1_{S'}$) der Spannbetonschwelle (1) in den Messpositionen gemäß der Gleichung:

$$\sin \vartheta_a/c_a = \sin \vartheta_b/c_b$$

bestimmt wird,

wobei $c_b$ die Schallgeschwindigkeit im Beton der Spannbetonschwelle (1) ist, $c_a$ die Schallgeschwindigkeit des Ankopplungsfluids ist, und $\vartheta_b$ ein Winkel der Oberflächennormalen der Oberfläche ($1_S$) der Spannbetonschwelle (1) bezüglich einer Grundfläche ($1_G$) der Spannbetonschwelle (1), einer Deckfläche ($1_D$) der Spannbetonschwelle (1) und/oder einer Ebene (E) ist, in der das durch den als Sender (3.1) konfigurierten Ultraschallprüfkopf des Paares von Ultraschallprüfköpfen (3) ausgesandte Ultraschallsignal die Spannbetonschwelle (1) in der jeweiligen Messposition die Spannbetonschwelle (1) durchlaufen soll.

11. Verfahren (1000) nach einem der vorstehenden Ansprüche,

wobei der jeweils definierte Winkel gemäß Schritt b. so gewählt ist, dass eine Ausbreitungsrichtung einer durch das Aussenden des Ultraschallsignals im Beton der Spannbetonschwelle generierten Transversalwelle mit einer im Beton der Spannbetonschwelle (1) generierten Longitudinalwelle weitestgehend zusammenfallen.

12. Verfahren (1000) nach einem der vorstehenden Ansprüche, wobei das Anordnen (1100) gemäß Schritt a. umfasst:

a1. Entnehmen der Spannbetonschwelle (1) aus dem Gleisbett;
a2. Reinigen der entnommenen Spannbetonschwelle (1) von oberflächlichen Verunreinigungen, und/oder
a3. Überführen der gereinigten Spannbetonschwelle (1) in einen Prüfbehälter (7) umfassend das Ankopplungsfluid (2).

13. Verfahren (1000) nach einem der vorstehenden Ansprüche, weiter umfassend:

Bestimmen des jeweiligen Schwellenwertes für die Ultraschalllaufzeit in der mindestens einen Messposition mittels einer Vergleichsmessung an einer Vergleichsspannbetonschwelle, typischerweise mittels Vergleichsmessungen an mehreren Vergleichsspannbetonschwellen und/oder mittels einer numerischen Simulation der Schallausbreitung in einem Modell für die Spannbetonschwelle.

14. System (100, 110, 120, 130) zur Prüfung einer Spannbetonschwelle (1) für deren Verwendbarkeit in einem Gleisbett, umfassend:

- zumindest ein Paar Ultraschallprüfköpfe (3, 3.1, 3.2...);
- ein Ankopplungsmedium;
- ein Bewegungsmittel (9), ausgewählt unter einem Prüfkopfbewegungsmittel (9) und einem Spannbetonschwellenbewegungsmittel (9); und
- eine Kontroll- und Steuereinheit, die eingerichtet ist, das zumindest eine Paar Ultraschallprüfköpfe (3, 3.1, 3.2...) und das Bewegungsmittel (9) so anzusteuern, dass das System ein Verfahren (1000) nach einem der vorhergehenden Ansprüche ausführt;
wobei ein Abstand zwischen in Längsrichtung der Spannbetonschwelle (1) zur Ermittlung der Folge von Ultraschalllaufzeiten eingenommenen jeweiligen Messpositionen in einem Bereich von 1 - 100 mm, bevorzugt in dem Bereich von 2 bis 10 mm liegt;
wobei optional das Bewegungsmittel (9) eingerichtet ist zur reproduzierbaren Positionierung des Paares Ultraschall-Prüfköpfe (3, 3.1, 3.2) an einer Vielzahl von Messpositionen, die in einer Ebene liegen, die orthogonal zu einer Durchschallungsachse ausgerichtet ist, und
wobei die Durchschallungsachse parallel einer Längsachse der Spannbetonschwelle (1) oder orthogonal zu einer Ebene, die durch die Längsachse der Spannbetonschwelle (1) definiert wird, orientiert ist.

15. Verwendung einer Messanordnung zur Bestimmung einer Ultraschalllaufzeit in einem Festkörper für eine Prüfung einer Spannbetonschwelle (1) auf deren Verwendbarkeit in einem Gleisbett,

wobei die Messanordnung ein System (100, 110, 120, 130) nach Anspruch 14 umfasst; und
wobei optional mindestens ein weiteres Paar Ultraschallprüfköpfe (3, 3.1, 3.2) so angeordnet ist, dass die Signale der verschiedenen Prüfkopfpaare (3) sich nicht gegenseitig beeinflussen und so eine gleichzeitige Prüfung der Spannbetonschwelle (1) in verschiedenen Messebenen (Messpositionen) möglich ist.

A

B

FIG. 1  C

EP 4 722 710 A1

110

A

B

FIG. 2

FIG. 3

FIG. 4

FIG. 5

FIG. 6

FIG. 7

1000

```
┌─────────────────┐
│                 │
│      1100       │
│                 │
└─────────────────┘
         │
         ▼
┌─────────────────┐
│                 │
│      1200       │
│                 │
└─────────────────┘
         │
         ▼
┌─────────────────┐
│                 │
│      1310       │
│                 │
└─────────────────┘
         │
         ▼
┌─────────────────┐         ┌─────────────────┐
│                 │         │                 │
│      1320       │         │      1600       │
│                 │         │                 │
└─────────────────┘         └─────────────────┘
         │
         ▼
┌─────────────────┐
│                 │
│      1400       │
│                 │
└─────────────────┘
         │
         ▼
┌─────────────────┐
│                 │
│      1700       │
│                 │
└─────────────────┘
```

FIG. 8

FIG. 9

FIG. 10

FIG. 11

FIG. 12

FIG. 13

$$\sin\vartheta_a / c_a = \sin\vartheta_b / c_b$$

11°

$\vartheta_a \sim 3°$

$\vartheta_b \sim 11°$

$1_S$

$c_a = 1480$ m/s
(Wasser)

$c_b = 5000$ m/s
(Beton)

$1_G$

$1$

$1_D$

$1_{S'}$

E

FIG. 14

Europäisches
Patentamt
European
Patent Office
Office européen
des brevets

# EUROPÄISCHER RECHERCHENBERICHT

## EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (IPC) |
|---|---|---|---|
| Y<br><br>A | US 5 275 051 A (DE BEER HARRY T [CA])<br>4. Januar 1994 (1994-01-04)<br>* Zusammenfassung; Abbildungen 2,4 *<br>* Spalte 1, Zeilen 5-9 *<br>* Spalte 3, Zeile 64 - Spalte 4, Zeile 16<br>*<br>* Spalte 8, Zeilen 3-21 *<br>* Spalte 11, Zeile 32 - Spalte 12, Zeile 40 *<br>----- | 1-3,6-8,<br>10-15<br>4,5,9 | INV.<br>G01N29/07<br>G01N29/04<br>G01N29/265<br>G01N29/28<br>G01N29/44<br><br>ADD.<br>G01N33/38 |
| Y | CN 112 986 124 A (UNIV HUNAN)<br>18. Juni 2021 (2021-06-18)<br>* Zusammenfassung; Abbildung 2 *<br>* Absätze [0002], [0004], [0010],<br>[0011], [0021] - [0025], [0037], [0043]<br>*<br>----- | 1-3,6-8,<br>10-15 | |
| Y | US 2017/370885 A1 (NA JEONG K [US] ET AL)<br>28. Dezember 2017 (2017-12-28)<br>* Zusammenfassung; Abbildungen 1,4A,4B *<br>* Absätze [0003], [0005], [0040] *<br>----- | 1-3,6-8,<br>10-15 | |
| Y | US 2013/014600 A1 (SARR DENNIS P [US] ET AL) 17. Januar 2013 (2013-01-17)<br>* Zusammenfassung; Abbildung 4 *<br>* Absätze [0018], [0040], [0043],<br>[0051], [0052] *<br>----- | 1-3,6-8,<br>10-15 | RECHERCHIERTE SACHGEBIETE (IPC)<br><br>G01N |
| Y | US 2012/216618 A1 (BLOOM JEFFREY A [US] ET AL) 30. August 2012 (2012-08-30)<br>* Zusammenfassung; Abbildungen 2,9 *<br>* Absätze [0004], [0010], [0025],<br>[0037], [0038], [0042], [0044] *<br>----- | 2 | |

Der vorliegende Recherchenbericht wurde für alle Patentansprüche erstellt

| Recherchenort | Abschlußdatum der Recherche | Prüfer |
|---|---|---|
| Den Haag | 12. Februar 2026 | de Jong, Frank |

EPO FORM 1503 03.82 (P04C03)

**ANHANG ZUM EUROPÄISCHEN RECHERCHENBERICHT
ÜBER DIE EUROPÄISCHE PATENTANMELDUNG NR.**

EP 25 20 3060

In diesem Anhang sind die Mitglieder der Patentfamilien der im obengenannten europäischen Recherchenbericht angeführten Patentdokumente angegeben.
Die Angaben über die Familienmitglieder entsprechen dem Stand der Datei des Europäischen Patentamts am
Diese Angaben dienen nur zur Unterrichtung und erfolgen ohne Gewähr.

12-02-2026

| Im Recherchenbericht angeführtes Patentdokument | Datum der Veröffentlichung | Mitglied(er) der Patentfamilie | Datum der Veröffentlichung |
|---|---|---|---|
| US 5275051 A | 04-01-1994 | AU 647649 B2 | 24-03-1994 |
| | | CA 2069971 A1 | 12-03-1993 |
| | | US 5275051 A | 04-01-1994 |
| CN 112986124 A | 18-06-2021 | KEINE | |
| US 2017370885 A1 | 28-12-2017 | US 2017370885 A1 | 28-12-2017 |
| | | WO 2017223499 A1 | 28-12-2017 |
| US 2013014600 A1 | 17-01-2013 | CA 2776441 A1 | 11-01-2013 |
| | | CN 102879479 A | 16-01-2013 |
| | | EP 2546640 A2 | 16-01-2013 |
| | | JP 5954779 B2 | 20-07-2016 |
| | | JP 2013040920 A | 28-02-2013 |
| | | KR 20130007964 A | 21-01-2013 |
| | | US 2013014600 A1 | 17-01-2013 |
| | | US 2013305849 A1 | 21-11-2013 |
| US 2012216618 A1 | 30-08-2012 | KEINE | |

EPO FORM P0461

Für nähere Einzelheiten zu diesem Anhang : siehe Amtsblatt des Europäischen Patentamts, Nr.12/82

**IN DER BESCHREIBUNG AUFGEFÜHRTE DOKUMENTE**

*Diese Liste der vom Anmelder aufgeführten Dokumente wurde ausschließlich zur Information des Lesers aufgenommen und ist nicht Bestandteil des europäischen Patentdokumentes. Sie wurde mit größter Sorgfalt zusammengestellt; das EPA übernimmt jedoch keinerlei Haftung für etwaige Fehler oder Auslassungen.*

**In der Beschreibung aufgeführte Patentdokumente**

- CA 2955105 A1

- KR 101027910 B1

**In der Beschreibung aufgeführte Nicht-Patentliteratur**

- Betonschwellen Feste Fahrbahn Fertigteiltragplatten Komponenten im Netz der Deutschen Bahn AG. Hrsg.: Betonschwellenindustrie e.V. 2017, vol. 2